# EUROPÄISCHE PATENTSCHRIFT

(11) **EP 2 804 639 B1**
(45) Veröffentlichungstag und Bekanntmachung des Hinweises auf die Patenterteilung: **02.05.2018**
(21) Anmeldenummer: 13708069.3
(22) Anmeldetag: 22.01.2013
(51) Int. Cl.: A61L 29/16, A61L 31/16, A61K 9/06, A01N 25/04, A61L 2/16, A61L 2/26, A61M 25/00

(54) **VERFAHREN UND APPLIKATOR ZUR PERIOPERATIVEN DESINFEKTION VON DURCH NICHT NATÜRLICHE ÖFFNUNGEN EINZUFÜHRENDEN MEDIZINISCHEN INSTRUMENTEN**
METHOD AND APPLICATOR FOR THE PERIOPERATIVE DISINFECTION OF MEDICAL INSTRUMENTS TO BE INSERTED THROUGH NON-NATURAL OPENINGS
PROCÉDÉ ET APPLICATEUR DE DÉSINFECTION PÉRI-OPÉRATOIRE D'INSTRUMENTS MÉDICAUX À INTRODUIRE PAR DES ORIFICES NON NATURELS

(30) Priorität: 22.01.2012 DE 102012001216
(43) Veröffentlichungstag der Anmeldung: 26.11.2014
(73) Patentinhaber: Margraf, Stefan, 60318 Frankfurt (DE)
(72) Erfinder: MARGRAF, Stefan, 60318 Frankfurt (DE); STANGE, Jan, 18057 Rostock (DE)
(74) Vertreter: Westphal, Mussgnug & Partner Patentanwälte mbB
(86) Internationale Anmeldenummer: PCT/DE2013/000039
(87) Internationale Veröffentlichungsnummer: WO 2013/107443

(56) Entgegenhaltungen:
- EP-A1- 1 982 696
- EP-A2- 0 930 065
- WO-A1-01/28515
- US-A1- 2003 144 362
- US-A1- 2004 127 598
- US-A1- 2008 169 209

## Beschreibung

Aus der Gruppe der durch medizinische Instrumente bedingten Sepsen nehmen kathetersepsisbedingte Erkrankungen und Todesfälle einen großen Raum ein und gehören zum Bereich der theoretisch vermeidbaren Krankenhausinfektionen im Rahmen der Behandlung einer anderen schweren Erkrankung von Patienten. Allein in Deutschland geht man von mehreren tausend Todesfällen sowie Kosten in Milliardenhöhe jährlich aus.

Angesichts des Ausmaßes und der Auswirkungen von Kathetersepsis ist es dringend geboten verbesserte Verfahren und Mittel zu entwickeln, um die mit Kathetersepsis assoziierten Infektionen und Todesfälle zu reduzieren.

Die Ursache des Problems der Kathetersepsis ist im Wesentlichen, dass bei der Katheteranlage nur die Hautoberfläche desinfiziert wird und kein Verfahren oder Zubereitung bekannt ist, auch den Stichkanal zu desinfizieren, aus dem die meisten Keime stammen, welche den Katheter kontaminieren. Tiefere Hautschichten mit Haarfollikel, Talg- oder Schweißdrüsen enthalten nämlich ebenfalls Keime, die vom Oberflächendesinfektionsmittel nicht erreicht werden. Insbesondere für das Problem der Verschleppung von Keimen aus tieferen Hautschichten ist bislang keine Lösung gefunden worden.

Die vorliegende Erfindung stellt Verfahren und Mittel erstmals bereit, den Übertritt von vermehrungsfähigen Keimen aus dem Stichkanal auf medizinische Geräte zu verhindern und so der Kathetersepsis entgegen zu wirken.

Bereits bekannt ist die Anwendung von antiseptisch wirkenden Chlorhexidin-Beschichtungen bei Kathetern. Die Katheter werden final schon während der Herstellung beschichtet. Die Beschichtung ist mit 30-40 USD Mehrkosten gegenüber unbeschichteten Kathetern kostenintensiv, steht nur für eine kleine Minderheit der Katheter zur Verfügung. Im Moment des Durchdringens der Haut ist der Katheter trocken, eine Beschichtung im trockenen Zustand ist jedoch unwirksam. Daher können trotz Beschichtung Biofilme mit Keimen auf dem Katheter entstehen.

Bekannt ist das Produkt Instillagel, ein dickflüssiges Gel zur Anwendung bei Katheterisierungen der Harnröhre. Es hat die Aufgabe der Bereitstellung einer örtlichen Betäubung (Lidocain), eines Gleitmittels und enthält Chlorhexidin in einer unwirksamen Dosierung (0,05%) in einer Cellulosegelbasis. Es ist zur Anwendung bei Gefäßkathetern weder vorgesehen noch zugelassen. Weiterhin bekannt ist das Produkt Tegaderm CHG, welches ein Pflaster zum Abdeckung der Insertionsstelle eines angelegten Katheters darstellt. Das Produkt enthält eine feste Gelplatte, aus der Chlorhexidin (2%) über einen längeren Zeitraum freigesetzt wird um eine Infektion der Hautstelle zu verhindern, aus der der Katheter ragt. Es ist damit weder in der Lage noch dafür vorgesehen eine Kontamination des Katheters während der Katheteranlage zu verhindern, welches zur Blutsepsis führen kann. Es wird auch erst nach einer Anlage eines Katheters verwendet.

Dokument US 5,015,228 offenbart ein Gelpflaster, welches vor Entnahme von Flüssigkeiten (Blut) oder vor dem Zuführen von Flüssigkeiten (Medikamenten) aus Gefäßen von Menschen die Hautoberfläche an der Einstichstelle sowie die Nadel sterilisieren soll. Dieses Gelpflaster enthält ebenfalls eine feste Gelplatte, in welcher sich Desinfektionsmittel befinden. Allerdings sind die in US 5,015,228 für die Desinfektion vorgesehenen Substanzen nur bedingt geeignet, da sie entweder zu viel Zeit benötigen um ihre desinfizierende Wirkung zu entfalten oder/und nicht in den Körper gelangen sollten (fehlende Verträglichkeit). Nachteilig an diesem Gelpflaster ist, dass es zudem das Auffinden von Venen erschweren würde, da es eine zwar dünne und durchsichtige, aber dennoch zusätzliche feste Schicht auf der Haut darstellt, die das Ertasten und Sehen der zu punktierenden Vene beeinträchtigt. Überdies könnte beim Durchstechen des Gels eventuell die Nadel durch eine Gelausstanzung verstopft werden.

Dokument DE 198 30 421A1 beschreibt eine Einrichtung zur Behandlung und Pflege des Randbereichs von künstlichen Körperöffnungen, bzw. dem Bereich zwischen Patientenhaut und eingeführten medizinischen Instrumenten (z.B. Katheter, Halteschrauben, Schläuche) als mögliche Eintrittsstelle für Keime. Die Einrichtung ist vorgesehen zur Verwendung nach der Anlage eines medizinischen Instruments.

Die Offenbarung besteht in einer auf der Haut klebenden Kunststoffscheibe als Abgrenzung der Eintrittsstelle (Wunde) gegen kontaminierende Einflüsse aus der Atmosphäre, sowie einer Öffnung für z.B. ein antiseptisches Gel oder eine Paste, welche die Haut um die Eintrittsstelle feucht hält und antiseptische Wirkstoffe enthalten kann, die eine desinfizierende Wirkung auf die Wunde haben. Die in DE 198 30 421 offenbarte Einrichtung dient zum Schutz nach einem chirurgischen Eingriff, um die Wundheilung zu unterstützen und nachträgliche Kontamination der Eintrittsstelle von außen (durch äußere Einflüsse) zu verhindern.

WO2000033895 offenbart Cysteinderivate wie N-Acetylcystein in Verbindung mit Antibiotika zur Therapie von Biofilmen in Kathetern.

In den USA (Jahresbasis) entstehen bei 15 Millionen Behandlungstagen unter Einsatz zentraler Venenkatheter (auch: central venous catheter (CVC) days) auf der Intensivstation 80.000 durch Katheter bedingte Infektionen im Blutstrom (catheter-related bloodstream infections (CRBSI)) auf Intensivstationen, also etwa 5,3 Infektionen pro 1000 CVC days. Dies entspricht geschätzt etwa 250.000 Infektionen des Blutstroms (auch blood stream infections (BSI)) aus allen Krankenhausabteilungen inkl. Intensivstationen. (siehe Guidelines for the Prevention of Intravascular Catheter-related Infections, CID 2011:52, O'Grady et al). Dies verursacht USD 20.000 (Warren, 2006) -33.000 (Mermel, 2000) Extrakosten pro Infektion, was sich auf 5-8MRD USD Gesamtkosten/Jahr summiert. Der menschliche Schaden beläuft sich auf 24300 Todesfälle pro Jahr (2006) (Wenzel R and Edmond M. N Engl J Med 2006;355:2781-2783). In Deutschland entstehen bei 4,7 Millionen CVC-Tagen auf Intensivstationen 12.000 durch Katheter verursachte Infektionen des Blutstroms (auch: catheter-related blood stream infections (CRBSI)), mit einer ansteigenden Tendenz zwischen den Jahren 2000 und 2008 (1,8; 2; 2,5 bzw. 2,6 /1000 CVC-Tagen (KISS, Petra Gastmeier)). Es ist jedoch zu vermuten, dass diese Zahlen unvollständig sind, da die Meldungen auf freiwilliger Basis erfolgen (persönliche Mitteilung Dr. Geffers, KISS). Kathetersepsis verursacht 6000-10000 Todesfälle pro Jahr in Deutschland.

In der Gruppe der endoskopischen Eingriffe sind allein die Arthroskopien mit einer Infektwahrscheinlichkeit von 0,2-0,4% behaftet. Auch hier sind Staphylococcen der Hautflora die häufigsten Verursacher.

Zu den bei Kathetersepsis am häufigsten auftretenden Keimen gehören Keime der normalen Hautflora wie Koagulase negativer Staphylococcus (häufigster Keim bei Zentralvenöser Katheter (ZVK)-Sepsis) und auch Enterococcus spp., Proteus spp., P. aeruginosa, Klebsiella spp., S. aureus, darunter MRSA, Enterobacter spp. und E.coli.

Die Haut ist nur oberflächlich desinfizierbar. Tiefere Schichten, auch gerade Haarfollikel mit Talg- oder Schweißdrüsen enthalten jedoch ebenfalls Keime, die vom Desinfektionsmittel nur unzureichend erreicht werden und somit bei einer Katheteranlage auf den Katheter gelangen. Im Wesentlichen sind aus diesem Grund beispielsweise etwa 10% aller Blutkulturen falsch negativ.

Die Übertragung von sehr wenigen Keimen ist im Zusammenhang mit Fremdkörpern wie Kathetern sehr gefährlich. Elek und Conen² demonstrierten 1957, dass lediglich 100 Staphylokokken (pyogenes), assoziiert an Fremdkörper (hier: Nahtmaterial), in der Haut Gesunder schwere Infektionen auslösen. Im Gegensatz dazu wurden ohne Fremdkörper mehr als 1 Mio. Keime vertragen. Dies zeigt, das sehr geringe Keimzahlen in Assoziation mit Fremdkörpern zu schweren Infekten führen können.

Überdies ist ein Operationssaal nicht steril, sondern nur keimarm. Aerogene, Tröpfchen- und Schmierinfektionen kommen daher vor und können einen Katheter kontaminieren.

Katheterisierungen werden heute aseptisch, in der Regel im OP, durchgeführt. Wesentlich hierbei ist die Desinfektion der Punktionstelle. Diese wird vorwiegend mit alkoholbasierten Desinfektionsmitteln durchgeführt, seltener mit wasserbasiertem PVP-lod. Es werden mit dieser Methode etwa 99% der Hautoberflächenkeime inaktiviert³.

Die alkoholbasierenden Desinfektionsmittel können über den Alkohol hinaus weitere desinfizierende Verbindungen wie Chlorhexidindigluconat (Im Folgenden "Chlorhexidin" genannt) oder Octenidinhydrochlorid (Im Folgenden "Octenidin" genannt) enthalten.

Antiinfektiöse Zusammensetzungen werden z.B. in WO01/28515, US2003/144362 und EP1982696 sowie in US2004/127598 offenbart. Weiterer Stand der Technik ist in US 2008/1 69209 und EP 0 930 065 offenbart.

Angesichts des Ausmaßes und der Auswirkungen von Kathetersepsis ist es dringend geboten verbesserte Verfahren und Mittel zu entwickeln, um die mit Kathetersepsis assoziierten Infektionen und Todesfälle zu reduzieren. Insbesondere für das Problem der Verschleppung von Keimen aus tieferen Hautschichten ist bislang keine Lösung gefunden worden. Ebenso fehlt bislang eine Lösung für das Problem, dass Katheter nach der Entnahme aus ihrer Verpackung bakteriell kontaminiert werden können und in diesem Zustand verabreicht werden.

Die vorliegende Erfindung stellt Mittel und Verfahren zur Verfügung, die Verschleppung von Keimen auch aus tieferen Hautschichten mittels in nicht natürliche Öffnungen einzubringender medizinischer Instrumente wie z.B. Kathetern signifikant zu verringern sowie auch die Dekontamination perioperativ kontaminierter medizinischer Instrumente wie z.B. Katheter zu verbessern. Überdies ermöglicht die vorliegende Erfindung die effektive Desinfektion eines durch die Einführung eines medizinischen Instruments in den Körper erzeugten Stichkanals.

Soweit nicht anders angegeben lassen sich für bestimmte Ausführungsformen beschriebene Parameter und Eigenschaften auf sämtliche anderen hier offenbarten Ausführungsformen übertragen. Angaben in % beziehen sich auf Gew.-%, soweit nicht anders angegeben

Hierin beschrieben ist ein Verfahren zur perioperativen Desinfektion eines in eine invasiv erzeugte Körperöffnung einzuführenden medizinischen Instruments, vorzugsweise eines Gefäßkatheters, umfassend Aufbringen einer vorzugsweise viskosen oder schaumartigen, mindestens eine antiinfektiöse Verbindung enthaltenden Zusammensetzung auf das medizinische Instrument vor Ingebrauchnahme.

Die Erfindung bezieht sich auf ein Verfahren zur perioperativen Desinfektion eines in eine invasiv erzeugte Körperöffnung einzuführenden medizinischen Instruments, vorzugsweise eines Gefäßkatheters, und/oder eines durch die Einführung eines medizinischen Instruments in den Körper erzeugten Stichkanals, unter Verwendung des erfindungsgemäßen Applikators, umfassend Aufbringen einer viskosen oder schaumartigen, mindestens eine antiinfektiöse Verbindung enthaltenden Zusammensetzung auf das medizinische Instrument vor Ingebrauchnahme, dadurch charakterisiert dass die Zusammensetzung ausgebildet ist, um nach Inkontaktbringen mit dem medizinischen Instrument filmartig auf dem medizinischen Instrument zu haften und dass mindestens ein Teil der filmartig haftenden Zusammensetzung bei Einführung des medizinischen Instruments in den Körper mindestens bis in die Epidermis gelangt.

Im Zusammenhang mit der vorliegenden Erfindung ist unter "perioperativ" der Zeitraum unmittelbar vor, während und unmittelbar nach einer Operation, insbesondere unmittelbar vor einer Operation oder unmittelbar vor Ingebrauchnahme eines medizinischen Instruments, z. B. vor Anlegen eines Katheters, insbesondere vor, während oder nach einer Operation, zu verstehen. Unmittelbar meint hier einen zeitlichen Abstand zum Eingriff, d. h. der Operation und/oder Ingebrauchnahme eines medizinischen Instruments, von weniger als 1 h, weniger als 45 min, weniger als 30 min, weniger als 20 min, weniger als 15 min, weniger als 10 min oder weniger als 5 min, weniger als 2 min oder weniger als 1 min. Bei einem Zeitraum unmittelbar vor Ingebrauchnahme eines medizinischen Instruments, z. B. während einer Operation, beziehen sich die obigen Zeitangaben demnach auf Zeitabstände vor der Ingebrauchnahme eines medizinischen Instruments auch im Rahmen einer Operation.

Eine invasiv erzeugte Körperöffnung ist eine durch einen chirurgischen Eingriff erzeugte, natürlicherweise nicht vorhandene Körperöffnung, üblicherweise zum Ermöglichen bzw. zum Zwecke der Durchführung eines chirurgischen Eingriffs im Körper. Beispiele solcher invasiv erzeugter Körperöffnungen sind solche zur Einführung von medizinischen Instrumenten in Blutgefäße (z. B. Katheter oder Venenverweilkanülen) oder von Endoskopen für minimalinvasive Eingriffe.

Ein in eine invasiv erzeugte (oder zu erzeugende) Körperöffnung einzuführendes medizinisches Instrument ist bevorzugt stabförmig (Querschnittsdurchmesser im Verhältnis zur Länge kleiner als 1/10) und kann auch flexibel sein. Die in der vorliegenden Erfindung zu verwendenden medizinischen Instrumente durchdringen bei Einführung in den Körper die Haut, welche hierfür gewöhnlich mit einer scharfen Schneide durchschnitten wird, wie zum Beispiel durch eine Kanüle. Beispielhafte erfindungsgemäße medizinische Instrumente schließen Gefäßkatheter, Peritonealkatheter, Elektroden, Shunts, Drainagen, Venenverweilkanülen, für die Blutentnahme bestimmte Kanülen, Ports und Pins (nach außen ragende Drähte eines Fixateurs) ein. Ebenso umfasst sind Rohrschaftinstrumente wie Endoskope, und Trokare, welche die Haut durchdringen, wie z.B. für die Arthroskopie, Laparoskopie und andere operative Verfahren. Insbesondere nicht von dem Begriff umfasste medizinische Instrumente sind solche, die nicht die Haut durchdringen, wie z.B. Blasenkatheter, Gastroskope, Koloskope oder Bronchoskope.

Katheter sind flexible oder starre Hohlkapillaren mit einem oder mehreren Lumina, mit mindestens einem Ein- und Ausgang, wahlweise mit einer Zuführung am distalen, d.h. körperabgewandten Ende. Gefäßkatheter umfassen sämtliche in der Medizin gebräuchlichen Katheter, wie z. B. solche zur Applikation von flüssigen Stoffen, zum Abfluss von Flüssigkeiten wie z. B. Blut oder für Gase (z. B. Shunts) sowie Herzkatheter, Venenverweilkatheter z. B. für V. jugularis oder V. subclavia.

Die Zusammensetzung wird filmartig auf den Katheter aufgetragen. Dabei ist die Viskosität der Zusammensetzung derart gewählt, dass sie als Film (geschlossener Film oder Film der die Oberfläche (annähernd) vollständig bedeckt) insbesondere im feuchten Zustand am Instrument haftet. Die Dicke der auf den Katheter aufgetragenen Schicht kann zwischen 10 µm und 500 µm liegen, kann aber auch dicker sein. Bei einer Schichtdicke ab 10µm wird eine ausreichende Menge der antiinfektiösen Verbindung aufgebracht um die antiinfektiöse Wirkung zu erzielen. Die Dicke der Schicht überschreitet bevorzugt nicht 3 mm, weiter bevorzugt nicht 2 mm. Bevorzugt wird eine Mindestschichtdicke von 0,1 mm erreicht. Hierbei soll die Beschichtung mindestens 80%, bevorzugt 90%, weiter bevorzugt mindestens 95% oder 100% der Oberfläche des medizinischen Instruments, die im Körper verbleiben wird, erfassen.

Eine erfindungsgemäße viskose Zusammensetzung ist so ausgelegt, dass sie, spätestens sobald sie auf das medizinische Instrument aufgebracht ist, weitestgehend in der aufgetragenen Form verbleibt. Die Zusammensetzung wird bevorzugt film- oder schichtartig auf das medizinische Instrument aufgetragen. In einer bevorzugten Ausführungsform ist die Viskosität der Zusammensetzung derartig, dass die Zusammensetzung als Film auf dem Katheter haftet, so lange keine Scherkräfte auf ihn einwirken. So ist die Zusammensetzung abwischbar.

Solche Eigenschaften lassen sich beispielsweise durch Versetzen einer Flüssigkeit, vorzugsweise einer wässrigen Flüssigkeit, mit einem Gelbildner bzw. ein Thixotrop oder Dickungsmittel erzielen. Beispielhaft dafür sind Bentonite, Kieselsäuren, Polyacrylsäuren (PAA, z. B. Carbomeren),Polyvinylpyrrolidone, Polyvinylalkohole (PVA), Polyacrylate, Cellulose und Cellulosederivaten wie Hydroxyethylcellulose, Na-Carboxymethylcellulose, Hydroxypropylmethylcellulose, Hydroxyethylcellulose, Xanthanen, Hypromellose, Gelatine, Pektin, Casein, Agar-Agar, Stärke, Traganth, Polyethylenglycol, natürliche oder synthetische Albumine sowie deren Mischungen, Hyaluronsäure, Polyethylenglycol, Lecithin, Glycerin, Glucose, Hydroxypropyl Methylcellulose, Propylenglycol oder/und Polysorbat 80, , Dexpanthenol und Polysaccharide (z.B. Carrageen). Vorzugsweise ist der Gelbildner ein Carbomer. Beispiele für Carbomeren umfassen Carbopol ETD2020, 1342, 1382 oder Carbomer 974P. Thixotrope umfassen beispielsweise Flüssigkeiten mit einem Anteil pyogener Kieselsäure (in medizinischen Qualitäten mit 100-600 m² Oberfläche pro g). In einer bevorzugten Ausführungsform ist der Gelbildner für den menschlichen Organismus ohne Komplikationen verträglich. Weiter bevorzugt umfasst er keine Cellulose oder ein Cellulosederivat.

Durch diese Eigenschaften der Zusammensetzung wird auch erreicht, dass sie bis zur Verwendung des medizinischen Instruments bzw. bei Verwendung eines Applikators zum Auftragen auf das medizinische Instrument, nicht abfließt.

Typischerweise weist die Zusammensetzung den Gelbildner in Mengen von 0,01 bis 20 Gew.-%, insbesondere 0,1 bis 10 Gew.-%, vorzugsweise 0,2 bis 5 Gew.-%, besonders bevorzugt 0,3 bis 2 Gew.-%, jeweils bezogen auf die Zusammensetzung, auf. Ist der Gelbildner PVA, so bewegt sich der Anteil an PVA in der Zusammensetzung zwischen 0,1 und 20 Gew.-%, bevorzugt zwischen 2 und 10 Gew.%, z.B. bei 3, 4, 5, 6, 7, 8 oder 9 %. Besonders bevorzugt ist ein Anteil von 5 Gew.%. Handelt es sich bei dem Gelbildner um PAA, z.B. ein Carbomer, so bewegen sich typische Konzentrationen an Gelbildner bei 0,1 bis 2 Gew.%, bevorzugt 0,2 bis 1,5 Gew.-%, z.B. 0,3, 0,4, 0,5, 0,6, 0,7, 0,8, 0,9, 1,0, 1,1, 1,2, 1,3 oder 1,4 Gew.%. Besonders bevorzugt ist ein Bereich zwischen 0,5 und 0,7 Gew.-%.

Im Allgemeinen weist die Zusammensetzung einen physiologisch verträglichen pH-Wert auf. Dies schließt pH-Werte im Bereich von 4 bis 8, bevorzugt 5 bis 8, besonders bevorzugt 5 bis 7 ein. Darüber hinaus kann es im Rahmen der vorliegenden Erfindung vorgesehen sein, dass die Zusammensetzung außerdem mindestens eine Säure und/oder Base, insbesondere zur Einstellung des pH-Werts, vorzugsweise zur Einstellung eines physiologisch verträglichen pH-Werts, bevorzugt zur Ausbildung eines Puffersystems, enthält. Die diesbezüglichen Puffersysteme sind dem Fachmann wohlbekannt, so dass es hierzu keiner weiteren Ausführungen bedarf.

Die erfindungsgemäße Zusammensetzung enthält mindestens eine antiinfektiöse Verbindung.

Der Begriff "antiinfektiös" beschreibt eine mindestens inaktivierende Wirkung einer Verbindung auf Mikroorganismen. Vorzugsweise weisen antiinfektiöse Verbindungen in wirksamer Dosis vernachlässigbare Nebenwirkungen auf, insbesondere bei Inkontaktbringen mit Blut oder Blutgefäßen, können jedoch auch in gewissem, tolerierbarem Grade toxisch für Körperzellen sein, wie zum Beispiel bei Ethanol. Antiinfektiöse Verbindungen oder Zusammensetzungen sind hinreichend bekannt.

Grundsätzlich sind alle antiinfektiösen Verbindungen zur Anwendung in der vorliegenden Erfindung geeignet, welche ein hohes Körperverträglichkeitsprofil aufweisen. Überdies liegt die mindestens eine antiinfektiöse Verbindung in einer Konzentration vor, die ihre antiinfektiöse Wirksamkeit sicherstellt.

In einer bevorzugten Ausführungsform ist die antiinfektiöse Zusammensetzung ausgewählt aus Chlorhexidin und Octenidin oder einer Kombination daraus.

Enthält die Zusammensetzung Octenidin (1,1 '-(1, 1 O-Decandiyl)bis[4-(octylamino)pyridinium]-dichlorid bzw. 1,1'-Decamethylen[(1,4-dihydro-4-octylimino)pyridinium]dichlorid C₃₆H₆₄N4Cl₂) bzw. dessen Salze, insbesondere Octenidindihydrochlorid, so sind diese üblicherweise in Mengen von 0,01 bis 2 Gew.-%, insbesondere 0,02 bis 1 Gew.-%, vorzugsweise 0,03 bis 0,5 Gew.-%, noch weiter bevorzugt 0,03-0,12% beispielsweise 0,05, 0,06, 0,07, 0,08, 0,09, 0,1, 0,11, 0,12%, jeweils bezogen auf die Zusammensetzungen enthalten.

Überraschenderweise hat sich gezeigt, daß entgegen der üblichen Konzentration von 0,1% auch eine Konzentration von 0,05% Octenidin ausreichend gut wirksam ist und den Vorteil einer noch geringeren Reizung aufweist. Ohne auf einen Wirkmechanismus festgelegt werden zu wollen, nehmen die Erfinder an, dass der Grund in einem geringeren Verteilungsvolumen in einer Gelformulierung bezogen auf die Gesamtmenge des Gels liegt.

Eine weitere bevorzugte Ausführungsform der antiinfektiösen Zusammensetzung enthält zusätzlich noch 2-Phenoxyethanol in einer Menge von 0,5 bis 4%, bevorzugt 2%.

Chlorhexidin kann in Mengen von 0,1-5%, bevorzugt 0,5 bis 2%, z.B. 1% oder 1,5% vorliegen. Eine bevorzugte antiinfektiöse Zusammensetzung umfasst Chlorhexidingluconat als antiinfektiöse Verbindung, welches typischerweise in einer Konzentration von 0,1 bis 5%, bevorzugt 0,5 bis 2%, z.B. 1% oder 1,5%, angewendet wird.

In einer weiteren bevorzugten Ausführungsform ist die antiinfektiöse Verbindung ausgewählt aus quaternären Ammoniumverbindungen wie z.B. Cetylpyridiniumchlorid (Konzentration zwischen 0,01 und 0,05%, bevorzugt 0,03%; kann auch zusammen mit 0,1% Hexetidin vorliegen), Cetylpyridiniumbromid, Cetyltrimethylammoniumbromid, oder Benzalkoniumchlorid (Konzentration bevorzugt 0,05%-0,5%), 2-Phenoxyethanol, Triclosan, Ethylalkohol, Isopropanol, Polyhexanid (bevorzugt 0,01-20%, weiter bevorzugt 0,05 bis 0,5%, weiter bevorzugt in Kombination mit etwa der gleichen Konzentrationen von Undecylenamidopropylbetain) Macrogolum oder einer Kombination daraus. In einer anderen bevorzugten Ausführungsform liegen die obigen Verbindungen zusätzlich zu Octenidin oder Chlorhexidin vor.

In einer weiteren bevorzugten Ausführungsform ist die antiinfektiöse Verbindung ausgewählt aus Bacitracin, Gramicidin, Polymyxin B, Neomycin, Fusidinsäure, Gentamycin, Erythromycin, Chloramphenicol, Tetracyclin (Minocyclin, Doxycyclin) oder einem Gyrase-Hemmer (Levofloxacin, Ciprofloxacin), Rifampin, Rifampicin, Lincosamid, Trimethoprim/Sulfamethoxacol, Vancomycin, Linezolid, Mupirocin oder einer Kombination daraus. Diese Wirkstoffe liegen üblicherweise in einer Menge von bis zu 2 Gew.-% vor. In einer anderen bevorzugten Ausführungsform liegen die obigen Verbindungen zusätzlich zu Octenidin oder Chlorhexidin vor.

In einer weiteren bevorzugten Ausführungsform enthält die Zusammensetzung weiterhin eine antiinfektiöse Verbindung ausgewählt aus Povidon-lod und Phenoxyethanol oder einer Kombination daraus.

Übliche Konzentrationen an Polyvinylpyrrolidone-lod=Povidon-lod= PVP-I umfassen 4-12%, insbesondere 5%. Besonders bevorzugte antiinfektiöse Verbindungen oder Mischungen daraus umfassen Octenidinhydrochlorid 0,03-0,12% allein oder mit 2-4% Phenoxyethanol, 0,5-5% Chlorhexidin, auch als Chlorhexidindigluconat, Gyrasehemmer wie Ofloxazin oder staphylokokkenspezifische Antibiotika wie Fusidinsäure, Rifampin und andere.

Die antiinfektiöse(n) Verbindungen können auch als bzw. in Liposomen vorliegen, welche Vorteile bei hydrophoben Oberflächen und bei Mischungsinkompabilitäten bieten.

Das erfindungsgemäße Verfahren führt zur Reduktion der Anzahl von Fällen von durch Einbringung medizinischer Instrumente in Blutgefäße bedingter Sepsis, insbesondere Kathetersepsis. Der Begriff "durch medizinische Instrumente bedingten Sepsis" bezieht sich auf septische Infektionen in Zusammenhang mit dem Einbringen von Fremdmaterialien in den Körper zu medizinischen Zwecken (durch medizinische Instrumente). Dies gilt insbesondere für medizinische Instrumente, die über mehrere Replikationszyklen von Pathogenen wie z.B. Staphylokokken im Körper des Patienten verbleiben, so das ein Biofilm und eine gefährliche, fremdkörperassoziierte, vorwiegend hämatogene Sepsis entstehen kann. Durch das erfindungsgemäße Verfahren wird nicht nur die Anzahl der Sepsis-bedingten Todesfälle reduziert, sondern auch die durch Sepsis bedingten Intensivliegezeiten. Ebenfalls nicht zu vernachlässigen ist die Entlastung der Ärzte, der Kliniken und des Gesundheitssystems im Allgemeinen. Ohne auf eine bestimmt wissenschaftliche Theorie eingeschränkt werden zu wollen, gehen die Erfinder davon aus, dass diese Ergebnisse aufgrund dreier mit dem erfindungsgemäßen Verfahren verbundener Effekte erreicht werden:
- **Infektiöse Keime aus tieferen Hautschichten,** welche der oberflächlichen Desinfektion nicht zugänglich sind, haften normalerweise beim Durchtritt durch die Haut am Katheter an. Durch den antiseptischen Film auf dem Katheter werden sie nun einerseits vermindert aufgenommen und zusätzlich durch die antiseptische Wirkung inaktiviert
- **Inaktivierung** von Keimen, die unmittelbar nach Entnahme des Katheters auf diesen gelangen können, zum Beispiel aus der Luft über Tröpfcheninfektion oder Berührungen mit unsterilen Oberflächen
- **Abwaschwirkung:** Erzielung einer Waschwirkung am medizinischen Instrument, indem die erfindungsgemäße Zusammensetzung filmartig auf das medizinische Instrument aufgetragen ist und beim Einschieben durch die Haut vom Katheter abgeschoben wird, so das am Durchtrittsort durch die Haut Keime vom medizinischen Instrument weg in die Zusammensetzung verlagert werden und sich die Menge des abgeschobenen Gels während des Durchschiebens durch die Haut am Eintrittsort erhöht.

In anderen Worten wird durch die Viskosität der Zusammensetzung erreicht, dass diese zum einen am medizinischen Instrument vor Einbringen in ein Blutgefäß verbleibt. Zum zweiten wird der aufgetragene Film beim Einbringen in das Blutgefäß durch die Haut reduziert ("abgeschoben") und egalisiert, da das medizinische Instrument, z. B. ein Katheter, durch den an der Einstichstelle an der Haut entstehenden Tropfen der Zusammensetzung durchgeschoben wird. Dies hat zusätzlich zur Auftragung auch noch mal einen "Wascheffekt". Dennoch ist die Zusammensetzung wirksam im Stichkanal, da kleine Mengen der Zusammensetzung in die Haut hineingebracht werden und damit in der Epidermis und teilweise Dermis wirksam sind und letztlich von den kollagenen Faserbündeln der Dermis zurückgehalten werden. In diesen Bereichen befinden sich normalerweise Keime, die sich bei Einführung eines medizinischen Instruments auf dieses abstreifen. Somit wird durch das erfindungsgemäße Verfahren zusätzlich eine Stichkanaldesinfektion erreicht.

Das erfindungsgemäße Verfahren kann auch bei Blutentnahmen angewendet werden. Blutkulturen sind etwa 10% aller Fälle falsch positiv. Dies verursacht erhebliche Kosten und verzögert dringend benötigte Diagnosen. Kanülen, bestimmt für Blutentnahmen zum Zweck des Anlegens von Blutkulturen, können mit dem Verfahren in vorteilhafter Weise vor Kontamination mit Keimen bewahrt werden um damit die Rate an falsch positiven Kulturen zu senken.

In einer bevorzugten Ausführungsform liegt die Zusammensetzung als Gel vor.

Unter dem Begriff "Gel" sind im Rahmen der vorliegenden Erfindung insbesondere gelierte Flüssigkeiten zu verstehen, welche mit Hilfe geeigneter Quellstoffe, synonym auch als Gelbildner bezeichnet, hergestellt werden können. Es handelt sich hierbei vorzugsweise um feindisperse Systeme mit mindestens einer festen und einer flüssigen Phase, wobei der Quellstoff bzw. der Gelbildner die feste Phase bildet. Im Rahmen der vorliegenden Erfindung sind unter der flüssigen Phase sowohl Lösungen als auch Dispersionen zu verstehen, wobei die Dispersionen ihrerseits wiederum als Fest-Flüssig-Dispersionen vorliegen können. Durch den Gelbildner wird in dem feindispersen System, aus welchem das Gel aufgebaut ist, ein Netzwerk gebildet, wodurch die Viskosität der Mischung beeinflusst wird, was oftmals als "Gelierung" bzw. "Eindickung" beobachtet werden kann. Gele können als viskoelastische Fluide beschrieben werden, d. h. die Fluideigenschaften eines Gels liegen folglich zwischen denen einer idealen Flüssigkeit und denen eines idealen Feststoffkörpers. Im Rahmen der Erfindung zu verwendende Gele sind weder reißnoch schneidbar.

Besonders leistungsfähige gelartige oder verdickte Substanzen bzw. Eigenschaften werden erhalten, wenn die erfindungsgemäße Zusammensetzung eine Brookfield-Viskositat von 10-100 (Brookfield, cP x 1000, 20 rpm) oder mit 1 cP = 1 mPa·s = 0.001 Pa·s ist die dynamische Viskosität 10000-100000 mPas aufweist. In einer bevorzugten Ausführungsform weist die Zusammensetzung eine dynamische Viskosität η (mPa·s) größer 1, bevorzugt η (mPa·s) größer 4, weiter bevorzugt größer 5 und am meisten bevorzugt größer 20 auf. Die Viskosität ist bevorzugt niedriger als 100 Pa·s. Ein bevorzugter Bereich für Gele, z. B. PAA Gele, insbesondere solche mit EDT 2020, bewegt sich zwischen 20 und 60 Pa·s.

Die erfindungsgemäße Zusammensetzung kann außerdem einen Farbstoff, zur optischen Kontrolle des Auftrags, enthalten.

Darüber hinaus können Stabilisatoren, z.B. solche, die stabilisierend auf die Gelgrundlage oder die mindestens antiinfektiöse Verbindung wirken, wie zum Beispiel EDTA (Ethylendiamintetraessigsäure), Ethylenglycol-bis(aminoethylether)-N,N'-tetraessigsäure (EGTA), Natriumgluconat oder andere in Konzentrationen von 0,0001-1 Gew.-% hinzugefügt sein. Auch Tenside, wie Polysorbat, Triton, Tween oder andere können in Konzentrationen 0,0001-0,1% vorliegen. Sie erhöhen die Benetzbarkeit von hydrophoben Oberflächen.

Weiterhin können Konservierungsmittel wie Methyl-4-hydroxybenzoat, Propyl-4-hydroxybenzoat zur Zusammensetzung hinzugefügt sein.

Weiterhin kann es im Rahmen der vorliegenden Erfindung vorgesehen sein, dass die erfindungsgemäße Zusammensetzung außerdem mindestens einen vorzugsweise mehrwertigen Alkohol enthalt. Der mehrwertige Alkohol dient einerseits als Feuchthaltemittel und wirkt unterstützend bei der Einstellung optimaler Viskositätseigenschaften der erfindungsgemäßen Zusammensetzung und fungiert andererseits als Trägerstoff, welcher die Stabilität der erfindungsgemäßen Zusammensetzungen zusätzlich erhöht. Beispielhafte Polyole sind ausgewählt aus Di-, Tri- und Tetraolen, z. B. Mannit oder Sorbit.

Im Rahmen der vorliegenden Erfindung ist es vorteilhaft, wenn der vorzugsweise mehrwertige Alkohol ausgewählt ist aus der Gruppe von Polyvinylalkoholen, Glycerin, Glykolen und (Poly)Alkylenglykolen sowie deren Kombinationen und Mischungen, vorzugsweise Alkylen- oder Polyalkylenglykolen, besonders bevorzugt Propylenglykol.

Wenn die erfindungsgemäße Zusammensetzung einen vorzugsweise mehrwertigen Alkohol aufweist, so kann der vorzugsweise mehrwertige Alkohol in Mengen von 10 bis 80 Gew.-%, insbesondere 15 bis 70 Gew.-%, bevorzugt 20 bis 60 Gew.-%, jeweils bezogen auf die Zusammensetzung, in der Zusammensetzung enthalten sein.

Weiterhin enthält die erfindungsgemäße Zusammensetzung im Allgemeinen außerdem Wasser, insbesondere in Mengen von mindestens 10 Gew.-%, insbesondere mindestens 20 Gew.-%, bevorzugt mindestens 30 Gew.-%, jeweils bezogen auf die Zusammensetzung.

In diesem Zusammenhang ist es bevorzugt, wenn die Zusammensetzung das Wasser in Mengen von 10 bis 99 Gew.-%, insbesondere 80 bis 85 Gew.-%, besonders bevorzugt 80 bis 99,5 Gew.-%, jeweils bezogen auf die Zusammensetzung, enthält.

Besonders stabile und leicht zu applizierende Zusammensetzungen werden erhalten, wenn die erfindungsgemäße Zusammensetzung ein Gemisch oder eine Mischung mindestens eines vorzugsweise mehrwertigen Alkohols und Wasser aufweist. Vorzugsweise ist der Anteil an Wasser, jeweils bezogen auf das Gemisch oder die Mischung, mindestens 10 Gew.-%, insbesondere mindestens 30 Gew.-%, bevorzugt mindestens 50 Gew.-%. Darüber hinaus ist es erfindungsgemäß besonders bevorzugt, wenn das gewichtsbezogene Verhältnis von Wasser zu Alkohol in der Zusammensetzung im Bereich von 9:1 bis 1:5, insbesondere 3:1 bis 1:3, bevorzugt 2:1 bis 1:2, besonders bevorzugt 1,5:1 bis 1:1,5, variiert.

Alternativ zu den obigen Ausführungsformen, z. B. ein Gel, kann die vorzugsweise viskose Zusammensetzung ein die obigen Kriterien erfüllendes, nichtwässriges, biokompatibles Fett oder Öl (z.B. Sojabohnenöl, Olivenöl) sein und insbesondere deren Mischungen (Emulsionen) mit Wasser.

Die Zusammensetzung kann auch schaumartig sein. Hierzu enthält sie mindestens eine Verbindung, die die Schaumbildung der Zusammensetzung unter geeigneten Bedingungen befördert (siehe unten). Geeignete Schaumbildner sind z.B., allein oder in Kombination miteinander, oberflächenaktive Verbindungen wie zum Beispiel Detergenzien (Tenside) (Polysorbat, Triton, Tween, Konzentrationen 1-0,1%) oder auch Proteine wie z.B. natürliches oder synthetisches Albumin (Konzentrationen 0,1 - 50%) oder/und Polyvinylalkohol mit einem wässrigen Lösungsmittel, welches zusätzlich ein Treibmittel enthält. Dieses kann gelöstes oder chemisch gebundenes Kohlendioxid, Stickstoff oder Butan sein.

Weiterhin kann mit einem solchen Zusatz ohne Treibmittel ein Flüssigkeitsschaum erzeugt werden, indem der Zusatz in einem offenporigen, flexiblen Feststoffschaum (z. B. Polyurethan) vorliegt, durch welchen bei mehrmaligem Zusammendrücken Luft unter Schaumbildung zur Flüssigkeit gemischt wird. Auch sind insbesondere PVA Gele besonders leicht zu schäumen.

Die antiinfektiöse Zusammensetzung wird mittels des nachstehenden erfindungsgemäßen Applikators aufgebracht.

Der Applikator ist bevorzugt ein steriles Einmal-Medizinprodukt, kann jedoch auch in wiederverwendbarer Form vorliegen, sofern seine Bestandteile aus einem desinfizierbaren und/oder sterilisierbaren Material bestehen.

In einer bevorzugten Ausführungsform liegt die Zusammensetzung als Polyacrylsäuregel, bevorzugt als Carbomer Gel vor, wobei der Polyacrylsäureanteil bevorzugt zwischen 0,1 und 2 Gew.-% beträgt, z.B. 0,4, 0,5, 0,6, 0,7, 0,8, 1, 1,5 Gew.-%.

Der Begriff Carbomer ist ein allgemeiner Begriff für homopolymere Polyacrylsäuren (PAA). Ein Carbomer ist überlicherweise als ein weißes Pulver in verschiedenen Molekülgrößen erhältlich und wird zum Beispiel als Emulsionsstabilisierer oder Verdickungsmittel verwendet. Z.B Carbomer 940, 947, 943 oder auch als Carbopol (Marke von Lubrizol).

Die zusätzliche Verwendung von Polyvinylpyrrolidone (PVP) hat sich als vorteilhaft herausgestellt wegen einer besseren Standfestigkeit (d. h. gleichbleibende Schicht trotz Krafteinflüssen wie zum Beispiel der Schwerkraft oder hydrophiler/hydrophober Wechselwirkung) des Films auf hydrophoben Oberflächen und wegen der überraschenderweise sehr verbesserten Einmischbarkeit von Octenidin oder Chlorhexidin in Zusammensetzungen, die z. B. Gele sind. Dazu wird das Octenidin bzw. Chlorhexidin zunächst in dem PVP gelöst und dann mit dem Gel gemischt. Der PVP Anteil kann zwischen 0,1 bis 6 % betragen. Somit umfasst die Zusammensetzung in einer weiteren bevorzugten Ausführungsform weiterhin zwischen 0,1 und 6 Gew.-% Polyvinylpyrrolidon, bevorzugt 0,1 bis 5 Gew.-%, weiter bevorzugt 0,5 bis 5 Gew.-%, weiter bevorzugt zwischen 1 und 3 Gew.-%, z.B. 2,1, 2,2, 2,3, 2,4 oder 2,5 Gew.-%. In einer bevorzugten Ausführungsform liegt die Zusammensetzung als PAA Gel, bevorzugt Carbomer Gel vor und umfasst Polyvinylpyrrolidon, z. B. in den oben genannten Konzentrationen.

PVP ist ein wasserlösliches Polymer des Monomers N-Vinylpyrrolidon und außerdem bekannt als Povidon, Polyvidon. Povidon wird meist mit Molekülgröße angegeben, z.B. Povidon K90, K30, K25, außerdem ist es kommerziell erhältlich als Kollidon® (BASF), Plasdone® (ISP).

In einer besonders bevorzugten Ausführungsform umfasst die Zusammensetzung PAA, z. B. Carbomer, oder PVA als Gelbildner, PVP sowie Octenidin und/oder Chlorhexidin, alles in den an anderer Stelle genannten Konzentrationsbereichen.

In einer weiteren besonders bevorzugten Ausführungsform umfasst die Zusammensetzung 0,2-1% Carbomer und 0,01-0,12 Octenidin. Z. B. 0,03-0,07% Carbomer, z.B. Carbopol ETD 2020 NF, und 0,04-0,1% Octenidin sowie 0,5-5% Polyvinylpyrrolidon K90 (M 360 000 g/mol).

In einer anderen bevorzugten Ausführungsform ist das in eine invasiv erzeugte Körperöffnung einzuführende medizinische Instrument zum längeren Verbleib im Körper vorgesehen. Hierbei umfasst der Ausdruck "längerer Verbleib" Zeitspannen von 0,5 Tagen bis drei Monate, typischerweise 3 -28 Tage, beispielsweise 8 bis 12 Tage, wie z.B. häufig bei Intensivpatienten.

Wieterhin beschrieben wird eine mindestens eine antiinfektiöse Verbindung enthaltende Zusammensetzung, dadurch charakterisiert dass die Zusammensetzung ausgebildet ist, um nach Inkontaktbringen mit einem perioperativ in eine invasiv erzeugte Körperöffnung einzuführenden medizinischen Instrument filmartig auf dem medizinischen Instrument zu haften und dass mindestens ein Teil der filmartig haftenden Zusammensetzung bei Einführung des medizinischen Instruments in den Körper mindestens bis in die Epidermis gelangt.

Die wie oben beschriebene Zusammensetzung kann zur perioperativen Desinfektion von in eine invasiv erzeugte Körperöffnung einzuführenden medizinischen Instrumenten, insbesondere Gefäßkathetern oder Endoskopen, zum Waschen von obigen medizinischen Instrumenten oder als anti-Haftmittel oder Gleitmittel an obigen medizinischen Instrumenten oder zur Desinfektion eines durch die Einführung eines medizinischen Instruments in den Körper erzeugten Stichkanals verwendet werden.

Überdies beschrieben ist ein in eine invasiv erzeugte Körperöffnung, insbesondere in Blutgefäße, einzuführendes medizinisches Instrument, wobei eine vorzugsweise viskose oder schaumartige, vorzugsweise eine aus Chlorhexidin und Octenidin ausgewählte, antiinfektiöse Verbindung, enthaltende Zusammensetzung an dem medizinischen Instrument haftet, dadurch gekennzeichnet, dass die Zusammensetzung ausgebildet ist, um nach Inkontaktbringen filmartig auf dem medizinischen Instrument zu haften und dass die filmartig haftende Zusammensetzung bei Einführung des medizinischen Instruments in den Körper mindestens bis in die Epidermis gelangt.

Ebenfalls Gegenstand der vorliegenden Erfindung ist ein Applikator zur perioperativen Desinfektion eines in eine invasiv erzeugte Körperöffnung einführbaren bzw. einzuführenden medizinischen Instruments, wobei der Applikator derart ausgebildet ist, dass eine, viskose oder schaumartige, mindestens eine antiinfektiöse Verbindung enthaltende Zusammensetzung durch eine Relativbewegung zwischen dem Applikator und dem medizinischen Instrument auf eine Oberfläche des medizinischen Instruments aufbringbar ist, dadurch gekennzeichnet, dass der Applikator eine Rückhaltevorrichtung für die antiinfektiöse Zusammensetzung aufweist, wobei der Applikator als im Wesentlichen hohl ausgebildetes Reservoir mit einem, bevorzugt in Bewegungsrichtung hinten liegenden, konisch zulaufenden Abschnitt ausgebildet ist.

Die Rückhaltevorrichtung befindet sich in einer bevorzugten Ausführungsform in Bewegungsrichtung hinten liegend am Applikator. In einer weiteren bevorzugten Ausführungsform befindet sich der konisch zulaufende Abschnitt in Bewegungsrichtung hinten liegend am Applikator.

Soweit nicht anders angegeben lassen sich für obige Ausführungsformen beschriebene Parameter und Eigenschaften auf sämtliche anderen hier offenbarten Ausführungsformen übertragen. Dies trifft insbesondere auf mit dem Applikator zu benutzende antiinfektiöse Zusammensetzung zu.

In einer bevorzugten Ausführungsform entspricht der konisch zulaufende Abschnitt des Applikators der Rückhaltevorrichtung.

Durch die Rückhaltevorrichtung wird erreicht, dass überschüssiges Gel zurückgehalten wird. Außerdem wird durch sie der Auftrag durch Geldurchtrittsöffnungen ermöglicht und der Auftrag weitestgehend egalisiert.

In einer weiter bevorzugten Ausführungsform kann eine weitere Rückhaltevorrichtung auch am in Bewegungsrichtung vorn liegenden Ende des Applikators vorhanden sein. Sie kann z.B. in Form einer, bevorzugt elastischen Membrane ausgebildet sein, die besagtes Ende abschließt, z.B. aus Kunststoff oder Metall in Form einer Folie. In der Rückhaltevorrichtung befindet sich eine Öffnung zum Durchtritt des Applikators.

In einer weiteren bevorzugten Ausführungsform weist der konisch zulaufende Abschnitt eine Mehrzahl von Schlitzen auf, die bevorzugt vom in Bewegungsrichtung hinten liegenden Ende des konischen Abschnitts ausgehend in Axialrichtung ausgebildet sind. Besteht der Applikator aus dem konische zulaufenden Abschnitt, so ist höchstens einer der Schlitze durchgehend, um zu gewährleisten, dass der Applikator in einem Stück vorliegt.

Der konisch zulaufende Abschnitt ist in einer weiteren bevorzugten Ausführungsform im Wesentlichen kegelstumpfförmig. Mit anderen Worten liegt keine Kegelspitze vor, der konisch zulaufende Abschnitt ist aber dennoch so ausgebildet, dass er endseitig, d.h. im Bereich einer Deckfläche des Kegelstumpfes derart verschlossen ist, dass die mindestens eine antiinfektiöse Verbindung enthaltende Zusammensetzung nicht von selbst, d.h. ohne äußere Einwirkung aus dem Applikator austritt.

Kegelstumpfförmige konisch zulaufende Abschnitten können in Bewegungsrichtung hinten liegend eine Öffnung aufweisen, die jedoch so ausgestaltet ist, dass die mindestens eine antiinfektiöse Verbindung enthaltende Zusammensetzung nicht von selbst, d.h. ohne äußere Einwirkung aus dem Applikator austritt. Der Durchmesser einer solchen Öffnung kann z.B. zwischen 0,5 und 4 mm betragen, z.B. ca. 0,8, ca. 0,9, ca. 1, ca. 1,1, ca. 1,2, ca.1,3 wie z. B, 1,35, ca. 1,4, ca. 1, oder ca. 1,6 mm.

In einer alternativen Ausführungsform ist der konisch zulaufende Abschnitt im Wesentlichen kegelförmig, d. h. weist eine Spitze auf. Diese Spitze ist bevorzugt nicht geschlossen, sondern so gestaltet, dass das medizinische Instrument beim Durchführen durch den Applikator an der Spitze austreten kann. Ist die Spitze doch geschlossen, so ist sie so ausgebildet, dass das medizinische Instrument sie beim Durchführen durch den Applikator leicht durchdringen kann. Muss das medizinische Instrument nicht durch den Applikator hindurchgeführt werden, sondern wird in ihn hineingeschoben und wieder herausgezogen, ist die Beschaffenheit der Spitze so, dass ein kurzfristiger Druck, der durch versehentliches Anstoßen des medizinischen Instruments an der Spitze aufgebaut wird, keine negativen Folgen für den Applikator hat.

Im Zusammenhang mit der Form des konisch zulaufenden Abschnitts meint der Begriff "im Wesentlichen" eine tendenzielle Verjüngung einer rundlichen Grundform in Bewegungsrichtung nach hinten. Mit anderen Worten kann die Mantelform des Kegels bzw. Kegelstumpfes sich auch ungleichmäßig, z.B. geschwungen oder in Wellenform, verjüngen.

In einer weiteren bevorzugten Ausführungsform umfasst der Applikator zwei hintereinander angeordnete konisch zulaufende Abschnitte mit unterschiedlichen Winkeln a, wobei der Winkel des in Bewegungsrichtung vorn liegenden konisch zulaufenden Bereichs kleiner ist als derjenige des in Bewegungsrichtung am Ende liegenden konisch zulaufenden Bereichs.

Der Applikator kann aus einem ersten Teil bestehen, welcher von einer Hand kontaktiert wird und einem zweiten, welcher als Träger oder Reservoir der Zusammensetzung fungiert.

Hierbei ist der Träger bzw. das Reservoir an dem ersten Teil befestigt. Der Applikator kann auch nur aus dem Träger bzw. Reservoir konstruiert sein, welcher dann selbst von der Hand kontaktiert wird. Teil, Träger oder Hand können auch teilweise oder alle als Mehrzahl gemeint sein.

Der Applikator weist, z.B. in einer Innenzone, wenigstens ein Reservoir für die antiinfektiöse Zusammensetzung auf. Das Reservoir ist derartig ausgelegt, dass es die Speisung der mit dem medizinischen Instrument in Kontakt kommenden Teile des Applikators mit obiger Zusammensetzung ermöglicht, so dass ein weitgehend gleichmäßiges Auftragen auf das medizinische Instrument gewährleistet ist.

Der Applikator ist so ausgebildet, dass die Zusammensetzung durch Schieben oder Ziehen oder sowohl durch Schieben als auch durch Ziehen des medizinischen Instruments durch den Applikator auf das medizinische Instrument, z. B. einen Katheter, aufgetragen werden kann, so dass die Zusammensetzung schicht- oder filmartig auf das medizinische Instrument aufgetragen wird.

Der Träger bzw. das Reservoir kann als offenporiger Schaum, als ein unter Druck stehender steriler Behälter oder, mindestens zum Teil, flexibler Behälter oder als zerdrückbarer Blister ausgebildet sein. Bei Vorliegen eines unter Druck stehenden Behälters kann die Zusammensetzung durch ein Ventil oder durch Aufstechen mittels eines Dornes freigesetzt werden. In einer bevorzugten Ausführungsform schäumt die Zusammensetzung dann durch Vorhandensein von gelöstem CO₂ oder N2 auf.

Der Träger kann auch als Reservoir an antiinfektiöser Zusammensetzung vorliegen, welches die Zusammensetzung umfasst, die bei ausreichender Viskosität bzw. ausreichend festem Schaum am vorgesehenen Platz verbleibt.

Der Applikator weist, bevorzugt in Bewegungsrichtung des medizinischen Instruments, eine Rückhaltevorrichtung für die antiinfektiöse Zusammensetzung auf. Die bevorzugte Ausführungsform, in der die Rückhaltevorrichtung in Bewegungsrichtung des medizinischen Instruments liegt, entspricht bezogen auf die relative Bewegungsrichtung des Applikators einer Anordnung am hinteren Ende des Applikators ist also in Bewegungsrichtung hinten liegend. Eine solche Rückhaltevorrichtung verhindert das Austreten von antiinfektiöser Zusammensetzung über den gewünschten Auftrag auf das medizinische Instrument hinaus, z. B. in Form von Hinausfließen oder -tropfen.

Die Rückhaltevorrichtung kann aus Kunststoff, beispielweise als elastische Folie, bevorzugt mit einer zentrischen Öffnung für das medizinische Instrument, ausgebildet sein. Die Rückhaltevorrichtung kann an geeigneter Stelle geschlitzt sein, so dass der vorliegende Applikator für medizinische Instrumente unterschiedlichen Durchmessers verwendbar ist.

In einer bevorzugten Ausführungsform weist die Rückhaltevorrichtung mindestens eine Öffnung auf. Die Öffnung kann sich z. B. an der Deckfläche eines im Wesentlichen kegelstumpfförmigen konisch zulaufenden Abschnitts befinden, der in dieser Ausführungsform die Rückhaltevorrichtung umfasst. Diese Ausführungsform ist insbesondere für Applikatoren geeignet, durch die ein medizinisches Instrument hindurchgeführt wird. Die Öffnung dient in diesem Fall als Austrittsstelle für das medizinische Instrument.

Alternativ kann die Rückhaltevorrichtung an beliebiger Stelle mindestens eine Öffnung aufweisen. Durch solche Öffnungen kann überschüssige Zusammensetzung entweichen, wenn ein medizinisches Instrument in einen Applikator hineingeschoben und wieder hinausgezogen wird.

In einer weiter bevorzugten Ausführungsform ist die mindestens eine Öffnung zusätzlich geschlitzt. Beispielsweise kann der Applikator seitlich einen in Axialrichtung durchgehenden Schlitz aufweisen. Dieser kann als Eintritts und/oder Austrittsöffnung für das medizinische Instrument dienen. Dabei kann das medizinische Instrument durch Auseinanderbewegen der durch den Schlitz am Applikator erzeugten Kanten erfolgen. Auseinanderbewegen kann entweder durch die Verwendung eines elastischen Materials für den Applikator oder durch ein an anderer Stelle in Axialrichtung verlaufendes Scharnier erfolgen. Der durchgehende Schlitz ermöglicht unterschiedliche Vorgehensweisen bei der Auftragung einer antiinfektiösen Substanz auf ein medizinisches Instrument.

Nach Einbringen des medizinischen Instruments über den durchgehenden Schlitz wird der zuvor mit Gel befüllte Applikator geschlossen, wobei er an beliebiger Position entlang des medizinischen Instruments angeordnet werden kann, und zu seinem freien Ende abgezogen, wodurch die Beschichtung erfolgt. Alternativ kann auch umgekehrt das medizinische Instrument über das in Bewegungsrichtung vorne liegende Ende des Applikators in den Applikator hineingesteckt und sekundär nach der Beschichtung durch Öffnen des Applikators am durchgehenden Schlitz wieder entnommen werden.

Je nach der Quelle und Ort der antiinfektiösen Zusammensetzung auf dem Applikator kann während der Verweildauer des medizinischen Instruments durch Bewegen (z. B. Ziehen oder Schieben) des medizinischen Instruments die Zusammensetzung auf das medizinische Instrument aufgetragen werden. Das medizinische Instrument kann danach auch mit dem Applikator fixiert und geführt werden, so dass es nicht mit den behandschuhten Händen berührt werden muss. Alternativ kann ein Applikator auch mit zwei Relativbewegungen verwendet werden und muss dann keinen durchgehenden Schlitz aufweisen. Hierbei wird der Katheter zunächst mit dem freien Ende am in Bewegungsrichtung vorne liegenden Ende des Applikators eingeführt und anschließend wieder herausgezogen.

In anderen Ausführungsformen enthält der Applikator keinen durchgehenden Schlitz. In diesen Ausführungsformen kann der konisch zulaufende Abschnitt eine Anzahl von Schlitzen enthalten, die jedoch nicht durchgehend sind.

In besonderen Ausführungsformen ist der konisch zulaufende Abschnitt mehrfach geschlitzt, so dass die Schlitze bevorzugt sternförmig verlaufen und eine rosettenartige Struktur bilden. Im Zentrum der zusammenlaufenden Schlitze ist bevorzugt die Öffnung angeordnet die dann mit den Schlitzen eine Rosette bildet. Durch die mehrfache Schlitzung und eine geeignete Materialstärke ist der Applikator in dem konisch zulaufenden Bereich bevorzugt elastisch. Die Schlitze sind bevorzugt vom in Bewegungsrichtung hinten liegenden Ende des konischen Abschnitts ausgehend in Axialrichtung ausgebildet. Weite bevorzug wird durch die Schlitze eine Anzahl flexibler Zungen gebildet. Durch die Schlitzung entsteht bevorzugt eine Mehrzahl von flexiblen Zungen, die als Abstreifelemente bzw. als Rückhalteelement wirken. Bevorzug wird das Material dieses Teils des Applikators so gewählt, dass sich die Zungen bei Hindurchführen eines medizinischen Instruments durch den Applikator an dieses anschmiegen, so dass dabei der im Applikator aufgetragene Film antiinfektiöser Zusammensetzung im Wesentlichen erhalten bleibt. Auch die Dicke der Zungen muss an diese Gegebenheiten angepasst werden. Daher liegt das Material der Zungen bevorzugt in elastischer Form vor und weist eine Wandstärke (Dicke von außen nach innen) von ca. 0,2 bis 1,2 mm, bevorzugt 0,3 bis 0,8 mm auf. Die Anzahl der Zungen bewegt sich üblicherweise zwischen 3 und 20, bevorzugt zwischen 5 und 10, z.B. 6, 7, 8 oder 9.

Der Applikator kann mit dem Gel vorgefüllt sein. Das den Katheter kontaktierende Gel wird durch die Relativbewegung vom Katheter aus dem Applikator gezogen und durch die Zungen zum Teil zurückgehalten (gestaut) aber durch die Schlitze auch nach außen getragen und es verbleibt ein Film oder Schicht auf dem Katheter.

In einer bevorzugten Ausführungsform schließt sich an den konischen Abschnitt in Bewegungsrichtung ein zylindrischer Abschnitt an. Dieser zylindrische Abschnitt kann in Bewegungsrichtung vorne liegend endseitig offen sein. Alternative ist der zylindrische Abschnitt in Bewegungsrichtung vorne liegend endseitig geschlossen und weist nur eine Öffnung auf, die geeignet ist, als Eintrittsstelle des medizinischen Instruments in den Applikator zu dienen. In einer solchen Ausführungsform kann eine elastische Folie über ebendiesem Ende liegen, die eine geeignete Öffnung aufweist.

Der zylindrische Abschnitt kann wie der konisch zulaufende Abschnitt als Reservoir für die antiinfektiöse Zusammensetzung ausgebildet sein.

Auch wenn der konisch zulaufende Abschnitt eine Beschichtung des medizinischen Instruments gewährleistet, kann der zylindrische Abschnitt die Beschichtung noch optimieren, insbesondere im Hinblick auf einen Auftrag des Films auf der vollständigen Länge des medizinischen Instruments bei längeren Instrumenten.

In einer weiter bevorzugten Ausführungsform weist der Applikator, bevorzugt an dem seitlich in Axialrichtung verlaufenden durchgehenden Schlitz, eine Einführhilfe für das medizinische Instrument auf. Beispielsweise kann die Einführhilfe darin bestehen, dass die Kanten des durchgehenden Schlitzes zumindest teilweise lippenartig nach außen gebogen und fortgesetzt sind. Alternativ kann die Einführhilfe in einer Erweiterung des durchgehenden Schlitzes bestehen, die sich am Applikator in Bewegungsrichtung vorne liegend befindet.

Um ein Austreten der antiinfektiösen Zusammensetzung im Bereich des durchgehenden Schlitzes zu verhindern kann in diesem Bereich wenigstens eine Dichtkante, bevorzugt zwei gegenüberliegende Dichtkanten vorgesehen sein. Die Dichtkanten können so angeordnet sein und den durchgehenden Schlitz derart verengen, dass ein Austreten der antiinfektiösen Zusammensetzung, z.B. aufgrund deren Oberflächenspannung, verhindert wird. Die Dichtkanten können dabei bspw. die Flanken des durchgehenden Schlitzes einander annähern, können aber auch einander berührend oder einander überlappend ausgebildet sein.

Der Applikator kann mit der Zusammensetzung vorbefüllt oder -beschichtet sein. Eine solche Anordnung kann sterilisiert in einer Verpackung vorliegen und erst kurz vor Anwendung an dem medizinischen Instrument der Verpackung entnommen werden.

Der Applikator kann an seiner Außenseite mindestens zwei Eingriffzonen aufweisen, die bevorzugt so dimensioniert sind, dass ein menschlicher Daumen oder Finger hineinpasst. Die Eingriffzonen können aus einem Kunststoff oder einem Elastomer vorgeformt sein, in die jeweils Daumen und Zeigefinger hineingesteckt werden können. Die Eingriffzonen können z.B. zylinderförmig oder taschenförmig ausgebildet sein. Die Eingriffzonen können auf der proximalen Seite miteinander verbunden sein.

Um eine Bewegung des Applikators entlang des medizinischen Instruments zu erleichtern kann in Bewegungsrichtung vorne liegend eine Zughilfe vorgesehen sein. Diese Zughilfe kann beispielsweise dadurch gebildet sein, dass an dem Applikator endseitig im Wesentlichen in Radialrichtung auseinanderstrebende flügelartige Fortsätze angeordnet sind, die einerseits ein Abrutschen in diese Richtung verhindern und anderseits auch als Ansatzpunkt zum Ziehen des Applikators dienen. Alternativ kann an dem Ende als Zughilfe auch ein umlaufender Kragen vorgesehen sein, der ebenfalls ein Abrutschen verhindert und ein Ziehen erleichtert. Der umlaufende Kragen kann ebenfalls geschlitzt ausgeführt sein, sodass ein seitliches Einsetzen des medizinischen Instrumentes bzw. ein Abnehmen des Applikators von einem medizinischen Instrument nach wie vor ermöglicht wird. In besonderen Ausführungsformen ist die Zughilfe so geformt, dass sie Eingriffzonen bildet.

Sowohl die flügelartigen Fortsätze als auch der umlaufende Kragen können zur Stabilisierung in Zugrichtung Verstärkungsstreben aufweisen, die ein Abknicken oder Verbiegen in Zugrichtung vermindern oder verhindern können.

Die Eingriffzonen können durch eine geeignete Oberflächenstruktur, bspw. eine Riffelung oder in Querrichtung verlaufende Rippen oder Noppen gekennzeichnet sein. Gleichzeitige kann durch die Oberflächenstruktur eine verbesserte Haptik erreicht und ein Abrutschen des Benutzers verhindert werden.

Die Rippen können beispielsweise in Form von Pfeilspitzen, d.h. mit jeweils zwei aufeinander zu laufenden Flanken, ausgebildet sein. Auf diese Weise kann durch die Rippen gleichzeitig die Zugrichtung des Applikators angezeigt werden.

Der Applikator ist bevorzugt ein steriles bzw. sterilisiertes Einmal-Medizinprodukt oder sterilisierbar und wieder verwendbar.

Der Applikator kann eine Aufbringzone bzw. Innenzone aufweisen, in der das medizinische Instrument mit der Zusammensetzung in Kontakt kommt. Bevorzugt weist er auch eine Verteilungszone auf, durch welche die Beschichtung egalisiert wird, so dass überschüssige Zusammensetzung zurückgehalten wird. Überdies weist der erfindungsgemäße Applikator eine Handhabungszone auf, mit der der Applikator händisch geführt werden kann.

Der Applikator kann aus weichem, flexiblem und auch elastischem Material bestehen z. B. Silikonkautschuk, Elastomer, Thermoplast oder ein geschäumtes Material. Der erfindungsgemäße Applikator ist ein einteiliges, steriles Produkt, bevorzugt ein Einmalprodukt, welches nach Öffnung der Verpackung entnommen wird. Die sterile antiinfektiöse Zusammensetzung kann auch erst während der Anwendung auf den Applikator aufgetragen oder dieser befüllt werden, zum Beispiel aus einer Spritze oder einem anderer (Einmal-) Behälter. Der Applikator, die Menge an Zusammensetzung und das Verfahren sollen sicherstellen, dass nicht mehr Zusammensetzung als zum Erreichen des gewünschten Effekts unbedingt notwendig in das (die) Lumen (Lumina) des medizinischen Instruments gelangt.

Die Abmessungen eines erfindungsgemäßen Applikators hängt von dem damit zu verwendendem medizinischen Instrument ab. Üblicherweise weist der Applikator eine Länge zwischen 30 mm und 80 mm auf, bevorzugt zwischen 40 und 70 mm. Besonders bevorzugt Ausführungsformen weisen eine Länge von 45 bis 65 mm auf.

Die Länge der einzelnen Abschnitte des Applikators, wie z.B. mindestens ein konisch zulaufender Abschnitt oder ein zylindrischer Abschnitt, richten sich ebenfalls nach den Erfordernissen des einzuführenden medizinischen Instruments. Ein in Bewegungsrichtung hinten befindlicher konisch zulaufender Abschnitt kann z.B. eine Länge zwischen 5 und 20 mm aufweisen, ein sich in Bewegungsrichtung vorn an einen zylindrischen Abschnitt anschließender Abschnitt ist gewöhnlich länger als ein sich in Bewegungsrichtung am Ende befindlicher konisch zulaufender Abschnitt und ist z.B. zwischen 20 und 60 mm lang, bevorzugt zwischen 25 und 45 mm, z.B. 30, 35 oder 40 mm.

Der Durchmesser eines erfindungsgemäßen Applikators bewegt sich gewöhnlich zwischen 8 und 30 mm, bevorzugt zwischen 10 und 20 mm. Z.B. weist ein Applikator, ohne Einbeziehung von eventuell vorhandenen Einführhilfen in die Abmessung, einen Durchmesser von 12, 14, 15 oder 17 mm auf.

Die Länge der sich im konisch zulaufenden Abschnitt befindenden Schlitze übersteigt bevorzugt nicht die Länge des konisch zulaufenden Abschnitts selbst, die Schlitze können jedoch in Einzelfällen auch in den folgenden konisch zulaufenden oder zylindrischen Abschnitt fortgeführt werden, solange sie sich nicht über die gesamte Länge des Applikators erstrecken

Überdies umfasst die Erfindung ein Kit umfassend den in der vorliegenden Anmeldung beschriebenen Applikator sowie eine oder mehrere oben beschriebenen Zusammensetzung(en). Der Applikator samt bereits eingefüllter Zusammensetzung kann überdies in einer Verpackung vorliegen. Diese Verpackung ist bevorzugt eine konturnahe Verpackung, z.B. eine Blisterverpackung, die am für das in Bewegungsrichtung des Applikators vorne liegende Ende einen Öffnungsmechanismus vorsieht. In einer Ausführungsform kann das Kit überdies ein medizinisches Instrument, bevorzugt einen Gefäßkatheter oder ein anderes oben beschriebenes medizinisches Instrument umfassen. Die Verpackung schützt den Applikator vor Verschmutzung, erhält ihn steril und verhindert überdies ein Austrocknen des Gels.

Der Applikator kann in einer Ausführungsform an beliebiger Stelle des medizinischen Instruments geöffnet und vom medizinischen Instrument entfernt werden, ohne den Applikator vollständig über das medizinische Instrument zu ziehen.

Der Applikator kann aus einem elastischen Material bestehen, welches z.B. geschäumt oder durch Spritzguss hergestellt sein kann.

In einer Ausführungsform kann der Applikator aus einem offenporigen Schaum bestehen und z. B. im Wesentlichen zylinderförmig sein. Dieser Zylinder kann die, bevorzugt viskose, Zusammensetzung enthalten und bis zur Mitte längsseits geschlitzt sein. Ein solcher Applikator kann sich vor Verwendung z.B. in einer Folie befinden, die längsseits offen ist oder durch eine präformierte Schwachstelle zu öffnen ist.

Eines oder beide Enden des Applikators können durch eine Elastomer- oder Folienscheibe abgeschlossen sein, aufweisend ein zentrales Loch in der Mitte sowie vom Loch bis zum Rand einen Einschnitt, so dass der Applikator über den Katheter gestülpt und zusammengedrückt werden kann, und beim Durchziehen des Katheters überschüssige Zusammensetzung zurückgehalten wird und durch die elastische Eigenschaft der Scheibe ein dünner Film auf dem medizinischen Instrument verbleibt.

Ferner bezieht sich die vorliegende Erfindung auf die Verwendung eines erfindungsgemäßen Applikators zum Aufbringen einer vorzugsweise viskösen, mindestens eine antiinfektiöse Verbindung enthaltenden Zusammensetzung auf ein in Blutgefäße einzuführendes medizinisches Instrument vor Ingebrauchnahme des medizinischen Instruments. In einer bevorzugten Ausführungsform entspricht die mindestens eine antiinfektiöse Verbindung enthaltende Zusammensetzung der erfindungsgemäßen Zusammensetzung.

Ausführungsbeispiele eines erfindungsgemäßen Applikators werden nachfolgend unter Bezugnahme auf die beigefügten Figuren eingehend erläutert. Es zeigen:

### Zeichnungen:

- Figur 1:: Seitenansicht eines Applikators (nicht erfindungsgemäß)
- Figur 2:: Darstellung eines ersten Ausführungsbeispiels in a. Seitenansicht und b. Draufsicht auf den konisch zulaufenden Abschnitt
- Figur 3:: a. Seitenansicht und b. Draufsicht des Ausführungsbeispiels aus Figur 2 mit eingeführtem medizinischen Instrument
- Figur 4:: perspektivische Darstellung eines zweiten Ausführungsbeispiels
- Figur 5:: perspektivische Darstellung eines dritten Ausführungsbeispiels
- Figur 6:: perspektivische Darstellung eines a. vierten und b. fünften Ausführungsbeispiels
- Figur 7:: perspektivische Darstellung eines sechsten Ausführungsbeispiels
- Figur 8:: a. Vorderansicht und b. Seitenansicht eines siebten Ausführungsbeispiels mit unterschiedlichen konisch zulaufenden Abschnitten
- Figur 9:: Ausführungsbeispiel mit passender Verpackung in a. offenem und b. geschlossenem Zustand

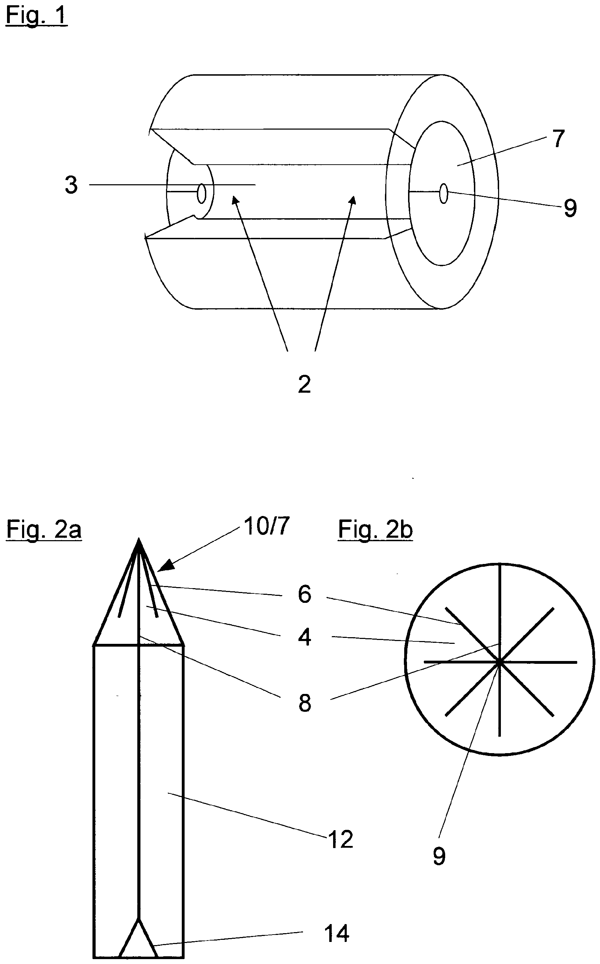

Figur 1 zeigt eine Seitenansicht eines Applikators (nicht erfindungsgemäß), in dem in der Innenzone 3 die antiseptische Zusammensetzung enthalten ist. Zusätzlich können geschlitzte Scheiben 7 als Rückhaltevorrichtung vorgesehen sein. Die Scheiben 7 weisen eine Öffnung 9 auf, die kleiner als der Durchmesser des medizinischen Instruments ist, um überschüssige Zusammensetzung vom medizinischen Instrument abzustreifen. Die Scheiben 7 können aus einem elastischen Folienmaterial sein.

Das medizinische Instrument kann dabei durch die Öffnung 9 geschoben werden, wobei aber durch die Schlitze der Scheiben 7 die Möglichkeit gegeben ist, den Applikator an einer beliebigen Stelle des Katheters aufzuschieben bzw. abzuziehen, ohne gezwungen zu sein, den Applikator zur Entfernung vom Katheter in die entgegengesetzte Richtung abzuziehen. Dies ist insbesondere dann vorteilhaft, wenn nur ein Teil des medizinischen Instruments in den Körper eingebracht werden soll und daher nur eine teilweise Behandlung notwendig ist.

Der Applikator kann komplett aus einem geschäumten oder gegossenen elastischen Material bestehen und auch ohne durchgehenden Schlitz gefertigt sein.

Figur 2 a zeigt eine Seitenansicht eines zweiten Ausführungsbeispiels eines Applikators 30. Der Applikator 30 ist im Wesentlichen aus einem im Wesentlichen zylindrisch ausgebildeten Abschnitt 12, der den Hauptteil eines Reservoirs 2 bildet, sowie einen daran anschließenden im Wesentlichen konisch zulaufenden Abschnitt 10 gebildet. Das Reservoir 2 enthält eine mindestens eine antiinfektiöse Verbindung umfassende Zusammensetzung und ist vorzugsweise damit gefüllt. Der konisch zulaufende Abschnitt 10 ist im vorliegenden Ausführungsbeispiel als Rückhaltevorrichtung 7 ausgebildet und von einer Spitze mit einer Öffnung 9 ausgehend in Draufsicht mit einer Mehrzahl sternförmig verlaufender Schlitze versehen. Durch die Schlitze 6 ist eine Mantelfläche des konisch zulaufenden Abschnitts 10 in eine Mehrzahl von Zungen 4 unterteilt. Durch eine geeignete Wahl der Anordnung und Anzahl der Schlitze sowie des für den konisch zulaufenden Abschnitt verwendeten Materials sind die Zungen 4 flexibel, so dass ein Hindurchtreten eines einzuführenden medizinischen Instruments möglich ist, auch wenn der Durchmesser des medizinischen Instruments den der Öffnung 9 überschreitet. Der Applikator 30 weist außerdem einen durchgehenden Schlitz 8 auf, der von der Öffnung 9 bis zu einem in Bewegungsrichtung vorne liegenden Ende des Applikators 30 verläuft. Der durchgehende Schlitz 8 ist in Bewegungsrichtung vorn liegend endseitig mit einer Einführhilfe 14 versehen. Die Einführhilfe ist im vorliegenden Ausführungsbeispiel als keilförmige Erweiterung des durchgehenden Schlitzes 8 ausgebildet. Es wird auf diese Weise ermöglicht, ein medizinischen Instrument, mit dem der Applikator 30 verwendet werden soll, mit Hilfe der Einführhilfe 14 leichter durch den durchgehenden Schlitz 8 ins Innere des Applikators 30 zu verbringen und den Applikator 30 anschließend in Bewegungsrichtung über das medizinische Instrument zu führen, wodurch eine Beschichtung mit der mindestens eine antiinfektiöse Verbindung enthaltenden Zusammensetzung erreicht wird.

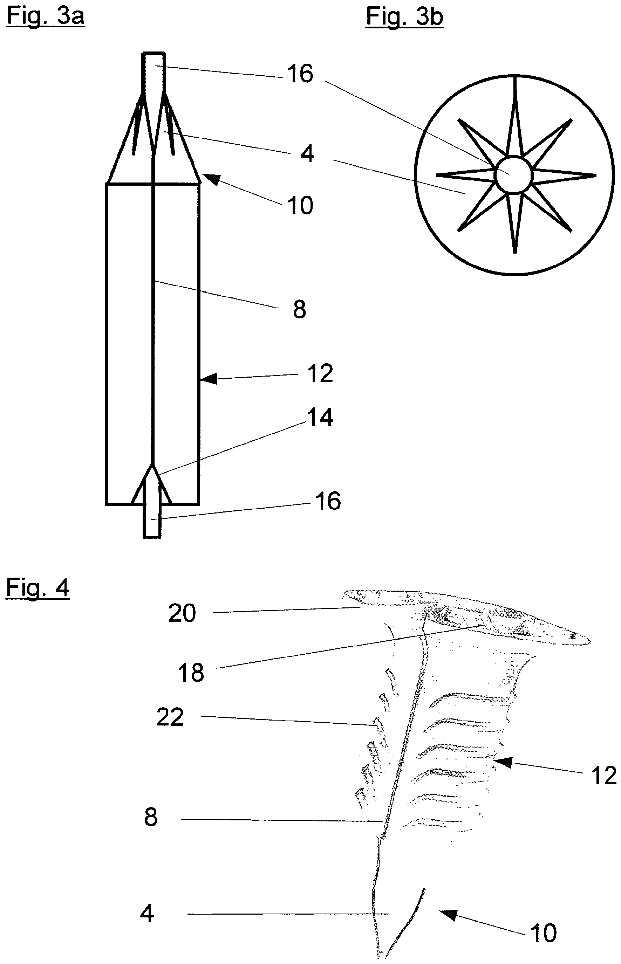

Figur 3 zeigt das in Figur 2 abgebildete Ausführungsbeispiel in Gebrauch. Ein medizinisches Instrument 16 befindet sich im Applikator 30. Die Zungen 4 am konisch zulaufenden Abschnitt 10 sind so weit auseinandergespreizt, dass das medizinische Instrument 16 durch den Applikator hindurchgeführt werden kann. Dabei schmiegen sich die Zungen 4 an das medizinische Instrument 16 so an, dass eine übermäßiger Austritt der im Reservoir 2 befindlichen mindestens eine antiinfektiöse Verbindung enthaltenden Zusammensetzung verhindert wird, jedoch trotzdem eine Beschichtung des medizinischen Instruments 16 mit der Zusammensetzung erfolgen kann.

Figur 4 zeigt eine perspektivische Darstellung eines dritten Ausführungsbeispiels eines Applikators 30. Der Applikator ist im Wesentlichen aus einem zylindrisch ausgebildeten Abschnitt 12, der den Hauptteil eines Reservoirs 2 bildet, sowie einen daran anschließenden konisch zulaufenden Abschnitt 10 gebildet. Das Reservoir 2 enthält eine mindestens eine antiinfektiöse Verbindung umfassende Zusammensetzung und ist vorzugsweise damit gefüllt. Der konisch zulaufende Abschnitt 10 ist im vorliegenden Ausführungsbeispiel als Rückhaltevorrichtung 7 ausgebildet und von einer Spitze mit einer Öffnung 9 ausgehend in Draufsicht mit einer Mehrzahl sternförmig verlaufender Schlitze versehen. Durch die Schlitze 6 ist eine Mantelfläche des konisch zulaufenden Abschnitts 10 in eine Mehrzahl von Zungen 4 unterteilt. Durch eine geeignete Wahl der Anordnung und Anzahl der Schlitze sowie des für den konisch zulaufenden Abschnitt verwendeten Materials sind die Zungen 4 flexibel, so dass ein Hindurchtreten eines einzuführenden medizinischen Instruments möglich ist, auch wenn der Durchmesser des medizinischen Instruments den der Öffnung 9 überschreitet. Der Applikator 30 weist außerdem einen durchgehenden Schlitz 8 auf, der von der Öffnung 9 bis zu einem in Bewegungsrichtung vorne liegenden Ende des Applikators 30 verläuft. In Bewegungsrichtung vorne liegend, d. h. am freien Ende des zylindrischen Abschnittes 12, ist der Applikator mit einer Zughilfe 20 versehen, die im vorliegenden Ausführungsbeispiel als umlaufender Kragen ausgestaltet ist. Der endseitig angeordnet umlaufende Kragen 20 ist zusätzlich mit radial verlaufenden Verstärkungsrippen 18 versehen, die ein Verbiegen der Zughilfe 20 in Bewegungsrichtung verhindern. Der durchgehende Schlitz 8 ist in Bewegungsrichtung vorn liegend endseitig mit einer Einführhilfe 14 versehen. Die Einführhilfe ist im vorliegenden Ausführungsbeispiel als keilförmige Erweiterung des durchgehenden Schlitzes 8 ausgebildet, die sich im freien Endbereich des zylindrischen Abschnittes radial nach außen erstreckt und eine keilförmige Aussparung in der Zughilfe 20 bildet. Es wird auf diese Weise ermöglicht, ein medizinisches Instrument, mit dem der Applikator 30 verwendet werden soll, mit Hilfe der Einführhilfe 14 leichter durch den durchgehenden Schlitz 8 ins Innere des Applikators 30 zu verbringen und den Applikator 30 anschließend in Bewegungsrichtung über das medizinische Instrument zu führen, wodurch eine Beschichtung mit der mindestens eine antiinfektiöse Verbindung enthaltenden Zusammensetzung erreicht wird. Der zylindrische Abschnitt 12 ist im vorliegenden Ausführungsbeispiel an seiner Oberflächen mit im Wesentlichen in Umfangsrichtung des Applikators verlaufenden Rippen versehen, die ein Greifen des Applikators 30, insbesondere mit behandschuhten Händen, vereinfachen, und ein Abrutschen beim Abziehen des Applikators in Bewegungsrichtung verhindern.

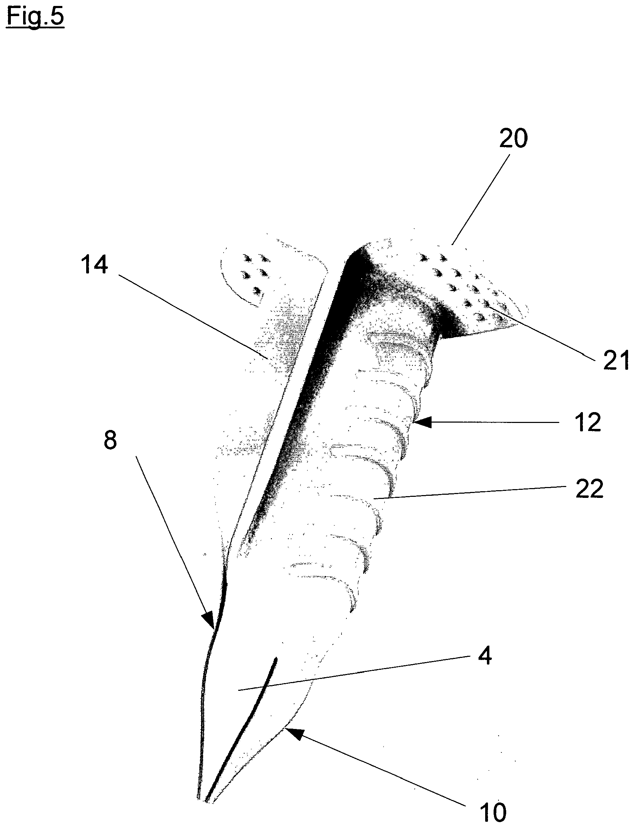

Figur 5 zeigt eine perspektivische Ansicht eines weiteren Ausführungsbeispiels des Applikators 30. Der Applikator 30 ist im Wesentlichen wie der Applikator 30 aus Figur 4 aufgebaut, wobei der durchgehende Schlitz 8 im Bereich des zylindrischen Abschnitts 12 vollständig mit einer Einführhilfe 14 versehen ist. Die Einführhilfe 14 ist im dargestellten Ausführungsbeispiel durch eine lippenartig auseinanderlaufende Fortsetzung der Flanken des durchgehenden Schlitzes 8 gebildet. Die Lippen setzen die Flanken des durchgehenden Schlitzes 8 im Wesentlichen in Radialrichtung fort und sind voneinander weglaufend beispielsweise in einem Winkel zwischen 20 und 90° angeordnet. Sie erleichtern so ein Einführen eines medizinischen Instruments durch den durchgehenden Schlitz. Die Lippen der Einführhilfe 14 gehen im vorliegenden Ausführungsbeispiel geschwunden über in die am freien Ende des zylindrischen Abschnitts vorgesehene Zughilfe 20 über. Die Zughilfe 20 ist in diesem Ausführungsbeispiel als in Radialrichtung auseinanderlaufendes Flügelpaar ausgebildet. An der dem zylindrischen Abschnitt zugewandten Seite weisen die Flügel der Zughilfe 20 eine Mehrzahl von Noppen 21 auf, die eine verbesserte Haptik bewirken.

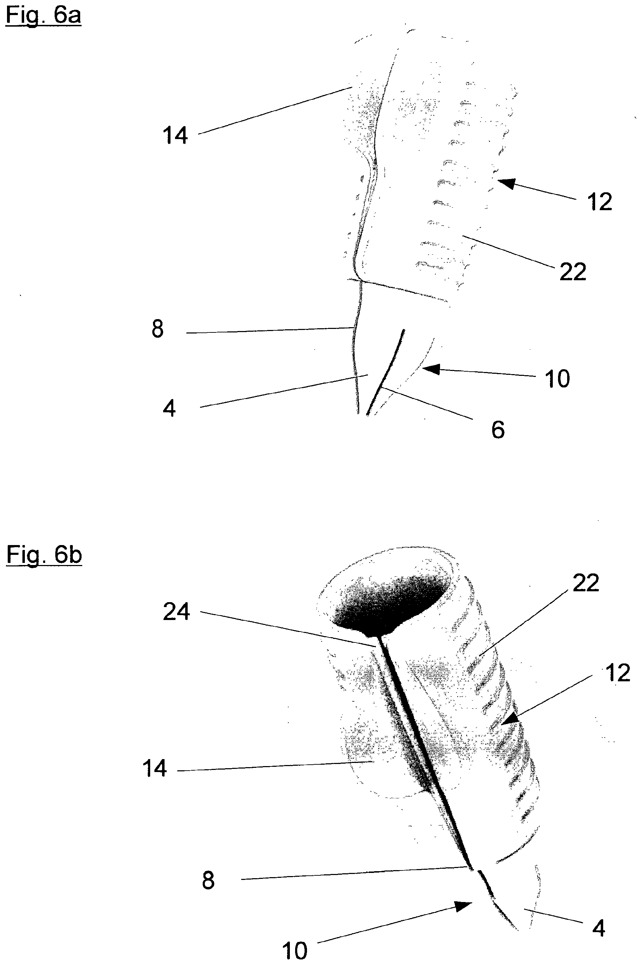

Figur 6 zeigt eine perspektivische Darstellung eines weiteren Ausführungsbeispiels. Bei diesem Ausführungsbeispiel ist keine Zughilfe vorgehsehen. Die Einführhilfe 14 ist im vorliegenden Ausführungsbeispiel zweigeteilt ausgestaltet, wobei sie in einem ersten Abschnitt des zylindrischen Abschnitts 12, der dem konisch zulaufenden Abschnitt 10 zugewandt ist, im Wesentlichen als ein lippenartiger, nach außen gebogener Fortsatz der Flanken des durchgehenden Schlitzes 8 ausgebildet ist. In einem zweiten Abschnitt des zylindrischen Abschnitts 12, der dem konisch zulaufenden Abschnitt abgewandt ist, ist die Einführhilfe 14 in Form von flügelartig ausgebildeten und auseinanderlaufenden angeformten Flächen ausgestaltet. Die Flächen der Einführhilfe 14 laufen so auseinander, dass einerseits die Einführung eines medizinischen Instruments erleichtert wird und andererseits ein manuelles Aufspreizen des Applikators an der Einführhilfe 14 ermöglicht wird. Dabei können die Flächen beispielsweise in einem Winkel zwischen 20 und 90° zueinander angeordnet sein.

Figur 6b zeigt eine weitere perspektivische Darstellung einer weiteren Variante des Applikators aus Figur 6a. In dieser Variante sind an dem durchgehenden Schlitz 8 im Bereich der Einführhilfe 14 zwei gegenüberliegenden Dichtkanten 24 vorgesehen, die den durchgehenden Schlitz im Bereich der Einführhilfe 14 verkleinern und somit ein Austreten der mindestens eine antiinfektiöse Verbindung enthaltenden Zusammensetzung weitgehend verhindern. Die Dichtkanten 24 können aus demselben Material sein wie der restliche Applikator oder aus einem anderen Material bestehend mit dem Applikator verbunden sein. In letzterem Falle kann das Material z.B. eine höhere Flexibilität oder Elastizität ausweisen als das des Applikators. Eine solche Ausführungsform kann beispielsweise in einem Zweikomponentenspritzgussverfahren hergestellt werden.

Bei Verwendung eines Zweikomponentenspritzgussverfahrens kann beispielsweise auch der konisch zulaufende Teil eines Applikators 30 aus einem anderen Material, beispielsweise erhöhter Flexibilität oder Elastizität bestehen, so dass ein verbessertes Anschmiegen der Zungen 4 an das medizinische Instrument ermöglicht wird.

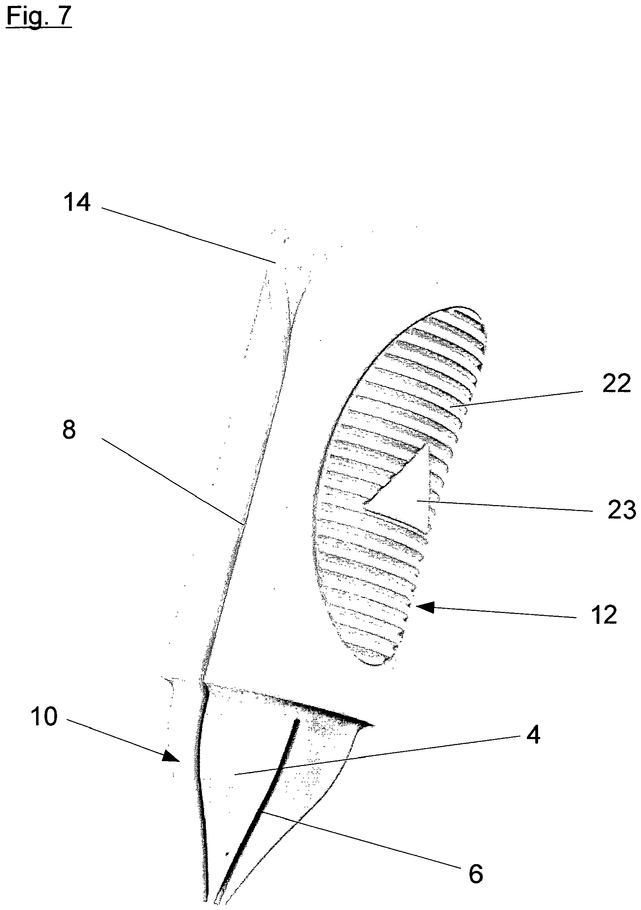

Figur 7 zeigt eine perspektivische Darstellung eines weiteren Ausführungsbeispiels eines Applikators 30, in der die Rippen 22 als Griffzone ausgebildet sind. Die Rippen verlaufen quer zur Längsrichtung des Applikators 30 und sind parallel angeordnet. In der Mitte der Griffzone befindet sich ein Richtungsanzeiger, der die Zugrichtung des Applikators anzeigt, wenn ein medizinisches Instrument hindurchgeführt wird. Die Rippen 22 sind in diesem Ausführungsbeispiel nicht als Anformungen an die Oberfläche des Applikators ausgebildet sondern durch einbringen einer Mehrzahl von parallel verlaufenden Nuten in die Oberfläche des Applikators gebildet.

Überdies verfügt der Applikator 30 im vorliegenden Ausführungsbeispiel über eine Einführhilfe, die als nach außen gebogene, am freien Ende des zylindrischen Abschnitts befindliche Kanten des durchgehenden Schlitzes ausgestaltet ist. In anderen Worten bildet ein sich an den Kanten befindender Teil der Wand des zylindrischen Abschnitts durch Aufbiegen die Einführhilfe 14.

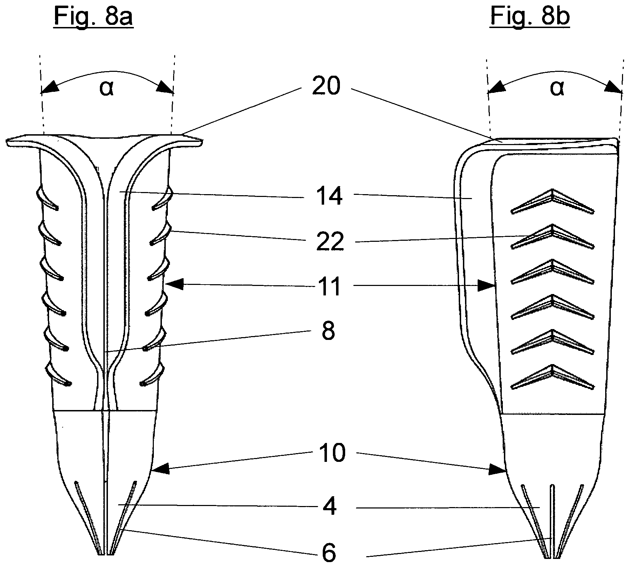

Figur 8 a zeigt die Vorderansicht, Figur 8b die Seitenansicht eines weiteren Ausführungsbeispiels eines Applikators 30. In diesem Ausführungsbeispiel besteht der Applikator aus zwei unterschiedlich stark konisch zulaufenden Abschnitten 10 und 11, wobei sich der in Bewegungsrichtung hinten befindliche konisch zulaufende Abschnitt 10 schneller verjüngt als der sich in Bewegungsrichtung vorne befindliche konisch zulaufende Abschnitt 11. Das heißt, dass ein Öffnungswinkel α bei dem sich in Bewegungsrichtung vorn befindlichen Ende kleiner ist als in dem sich in Bewegungsrichtung hinten befindlichen konisch zulaufenden Abschnitt. Bei dem in Bewegungsrichtung vorne liegenden konisch zulaufenden Abschnitt beträgt der Öffnungswinkel bspw. 6°. Dadurch, dass auch der in Bewegungsrichtung vorne liegende Abschnitt konisch ausgebildet ist, wird eine zusätzliche Führung bzw. Zentrierung eines medizinischen Instruments, mit dem der Applikator verwendet wird, erreicht. Ferner wird eine Befüllung des Applikators, sofern dieser nicht vorbefüllt ist, mit der wenigstens eine antiinfektöse Verbindung enthaltenden Substanz erleichtert.

Ein Übergang zwischen dem sich stärker verjüngend ausgebildeten Abschnitt und dem zweiten konischen Abschnitt ist durch Anpassung der Durchmesser im Übergangsbereich gebildet. Es kann entweder eine abschnittsweise oder eine stetige Anpassung des Öffnungswinkels alpha erfolgen.

An der engsten Stelle des sich in Bewegungsrichtung vorne befindlichen Abschnitts ist der sich in Bewegungsrichtung hinten befindlichen konisch zulaufende Abschnitt an seiner weitesten Stelle angeformt.

In dieser Ausführungsform sind die Rippen 22 als zwei Schenkel eines Dreieckes ausgestaltet, die auf diese Weise die Bewegungsrichtung des Applikators anzeigen können.

Die Einführhilfe 14 und die Zughilfe 20 sind ineinander übergehend ausgebildet und setzen an dem durchgehenden Schlitz 8 sowie dem freien Ende des Applikators schalartig an.

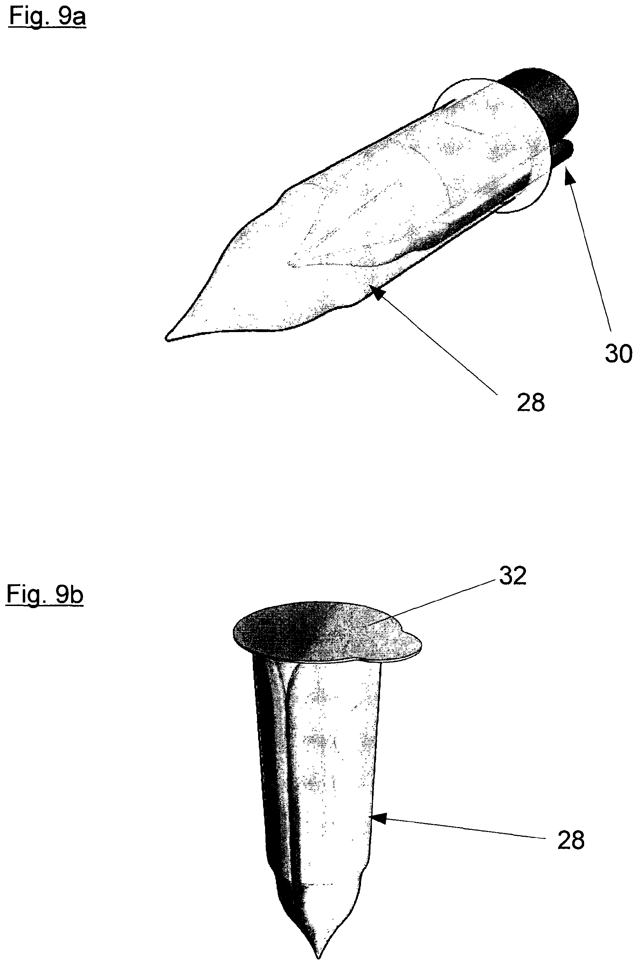

Figur 9 zeigt eine Ausführungsform des Applikators 30 in Verbindung mit einer Verpackung 28 Die Verpackung 28 weist einen Öffnungsmechanismus 32 auf, hier in Form einer abziehbaren flexiblen Folie, z.B. aus Metall oder Kunststoff.

Das Material des Applikators ist bevorzugt elastisch. Verschiedene Ausführungsformen des Applikators können zwei Eingriffzonen 1 aufweisen, in die Daumen und/oder Finger eingeführt werden können, um den Applikator zu halten und/oder um einen gegen die Mitte gerichteten Druck auszuüben. Zwischen den beiden Trägern 2 bzw. den beiden Trägern oder Reservoiren 2 befindet sich ein Hohlraum 3, in den ein in eine invasiv erzeugte Körperöffnung einzuführendes medizinisches Instrument, bspw. ein Endoskop, ein Gefäßkatheter oder eine Elektrode der Länge nach eingeführt werden kann. Sobald sich das medizinische Instrument, z.B. der Gefäßkatheter oder die Elektrode, zumindest zum Teil in dem Hohlraum 3 befindet, wird entweder, bedingt durch die Dicke des Katheters oder der Elektrode direkt Kontakt mit dem Träger 2 oder Reservoir 2 hergestellt oder dieser durch nach innen gerichtete Druckausübung ausgehend von den Eingriffzonen 1 hergestellt. Durch Schieben oder Ziehen des Katheters oder der Elektrode durch den Hohlraum 3 bei ausreichend hohem Druck kann eine gleichmäßige Beschichtung des Katheters oder der Elektrode mit einem Film aus der Zusammensetzung erreicht werden.

In der Innenzone bzw. dem Hohlraum 3 ist die antiseptische Zusammensetzung aufgetragen oder eingefüllt. Ein Katheter oder eine Elektrode kann entweder genau den Durchmesser der Innenzone 3 oder auch einen größeren oder geringeren Durchmesser aufweisen. Bei einem größeren Durchmesser wird das elastische Material des Applikators derartig gedehnt, dass das medizinische Instrument, z.B. der Katheter oder die Elektrode, in die Innenzone 3 passt. Durch Drehen des Katheters oder der Elektrode kann eine gleichmäßige filmartige Beschichtung erreicht werden. Bei einem geringeren Durchmesser kann von außen Druck ausgeübt werden, der zum Zusammendrücken der Innenzone 3 führt, so dass der Katheter oder die Elektrode vollständig beschichtet werden kann. Der Druck kann von einer Oberseite 5 und/oder einer Unterseite 6 des Applikators aus ausgeübt werden, entweder durch Ausübung an den jeweiligen Außenseiten oder durch Eingriffzonen, die an der figurabgewandten Seite in dem Applikator angeordnet sein können.

Zwischen 200 und 5000µl der Zusammensetzung sollten in der Regel ausreichen. Die Zusammensetzung kann hierbei in einem Träger 2 aus einem Schaumwerkstoff oder anderem Kunststoff gebrauchsfertig vorliegen. Der Träger kann auch aus einem elastischen Werkstoff aus Fasern gebildet werden. Der gesamte Applikator kann auch aus einem einzigen Werkstoff gefertigt werden, zum Beispiel aus einem, mindestens partiell, offenporigen Schaum.

### Beispiele

### Beispiel 1: Katheterkontamination bei künstlich infizierter, frischer Haut mit und ohne Behandlung mit erfindungsgemäßer Zusammensetzung

### Gelzubereitung:

A) Octenidin Gel 0,2%
B) Gelgrundlage: Carbomer 1% (hier: Cornegel: (Corneregel Dexpanthenol 5% in Carbomer 40-60000 MPas(Bausch&Lomb, Dr. Mann Ph))) Viskosität 50000 mPa s, pH 5,5 mit NaOH eingestellt, Dexpanthenol Zusatz 5%.

Schweinehaut mit muskulärem Hautanhang wurde mit Wasser und Seife gewaschen. Anschließend wurde das Gewebe unter der Haut so entfernt, dass die Unterseite steril war. Die Dicke des Präparats betrug 3-6 mm. Mit etwa 200 µl Bakterienkonzentrat (Staphylococcus epidermidis spp., gewonnen von frischer Schweinehaut (Minipig)) (ca. 0,5-1 x 10⁵ Koloniebildende Einheiten pro ml)) wurde Oberhaut mit einer Größe von etwa 10x10cm kontaminiert, die Haut anschließend 30min in einer Kulturschale bei 37 °C inkubiert. Anschließend wurden die verschiedenen Gele mit einem Applikator auf Braunülen aufgebracht und unmittelbar danach mit diesen die Haut, unter mehrmaligem Vor- und Zurückbewegen im Stichkanal, durchstochen. Die Kanüle wurde herausgezogen, der Katheter auf der Rückseite der Haut steril abgeschnitten, der Katheter auf Blut-Agar aufgebracht und eine Maki-roll⁶, mit etwa 20 leichten Impressionen in den Agar durch gleichzeitiges Beklopfen des Katheters während des Rollens auf dem Agar, ausgeführt.

Anschließend wurde alles in den Brutschrank gegeben und inkubiert und die Kolonien täglich gezählt.

Ergebnis:

| | | Kolonien nach 1 Tag | Mittelwert |
|---|---|---|---|
| 1 | Katheter ohne Gel | 1271 | 831 |
| 2 | Katheter ohne Gel | 211 | |
| 3 | Katheter ohne Gel | 492 | |
| 4 | Katheter ohne Gel | 1561 | |
| 5 | Katheter ohne Gel | 1012 | |
| 6 | Katheter ohne Gel | 648 | |
| 7 | Katheter ohne Gel | 624 | |
| 8 | Katheter mit Cornegel-Octenisept (CGO) 0,2% | 12 | 58 |
| 9 | Katheter mit Cornegel-Octenisept (CGO) 0,2% | 9 | |
| 10 | Katheter mit Cornegel-Octenisept (CGO) 0,2% | 72 | |
| 11 | Katheter mit Cornegel-Octenisept (CGO) 0,2% | 16 | |
| 12 | Katheter mit Cornegel-Octenisept (CGO) 0,2% Sehr dünner Hautabschnitt unter 1mm | 180 | |
| 13 | Katheter mit Cornegel-Octenisept (CGO) 0,2% | 34 | |
| 14 | Katheter mit Cornegel-Octenisept (CGO) 0,2% | 82 | |
| 15 | Abklatsch oben | >3000 | |
| 16 | Abklatsch Unterhaut nach Versuch | 248 | |

Die Kolonien wurden mit Hilfe einer Software (cell explorer 2000) der Fa. BioSciTec, Frankfurt, ausgezählt.

Wie aus obiger Tabelle ersichtlich, konnte eine 90%ige Reduktion von Keimen erreicht werden (von 831 auf 58).

### Ausführungsbeispiel 2: Katheterkontamination bei künstlich infizierter frischer Haut mit und ohne Behandlung mit erfindungsgemäßer Zusammensetzung

Testen eines 0,6% ETD2020 + 2,3% PVP + 0,05% OctenidinGels am Schweinehautmodell

### Gelherstellung von 0,6% ETD2020 + 2,3% PVP + 0,05% Octenidin

- 1,98g ETD2020 + 300ml dH2O (0,66%)
- bei 60°C unter Rühren quellen (> 1h)
- mit NaOH pH:6,3 einstellen
23% PVP + 66,5% dH2O + 0,5% Octenidin(di)hydrochlorid herstellen
10% der PVP/Octenidin-Mischung in 0,66% Gel einrühren = **0,6% ETD2020 + 2,3% PVP + 0,05% Octenidin**

Homogene Mischung, völlig klar, keinerlei Partikel zu erkennen.

| **Zutaten Gel** | **Hersteller** |
|---|---|
| Carbopol® ETD2020 NF Polymer | Lubrizol |
| Natriumhydroxid | ROTH |
| Octenidin(di)hydrochlorid | Caelo |
| Polyvinylpyrrolidon K90, M: 360 000 g/mol | ROTH |
| | |
| bidestilliertes Wasser | |

### Durchführung des Experiments/Ergebnisse:

Etwa 5x6cm Haut wurden aus der Leistengegend eines Schweins 10min nach ableben entnommen.

Am Versuchstag wurde die Haut zuerst gewaschen (mit Wasser und Seife) und dann mit etwa 400 **µl** Bakterienkonzentrat von Staph. epidermidis V1815 (1x10⁶/µl) kontaminiert, indem das Konzentrat mit dem Finger in die Haut eingerieben wurde.

Das Ganze kam dann für **4h** in den Brutschrank bei 37°C (mit Parafilm überdeckt).

Nach dieser Zeit wurde 6x mit verschiedenen Kanülen durch die Haut gestochen (Kontrolle 1-6), steril abgeschnitten und auf Blutagar gebracht (durch eine intensive Maki-roll, dh. mind. 4 mal über die Platte rollen mit etwa 50 leichten Impressionen durch Beklopfen).

Anschließend wurde die gleiche Prozedur 6x mit Kanülen mit Gelbeschichtung durchgeführt (Gel 7-12) und danach ein Abklatsch der Haut gemacht.

Die Platten wurden dann für 16h im Brutschrank bei 37°C inkubiert.

| **Gel 0,05%** | **Kontrolle** | |
|---|---|---|
| 336 | 7000 | |
| 23 | 8000 | |
| 147 | 7000 | |
| 212 | 8000 | |
| 157 | 8000 | |
| 460 | 5000 | |
| **223** | **7167** | **MW** |
| **154** | **1169** | **stabw** |
| **3,10%** | **100,00%** | **%** |

### Fazit/Zusammenfassung:

Die Platte wurde mit dem Zählprogramm (cell explorer) ausgezählt.

Ergebnis:
Durch Verwendung des Gels wurde die Keimzahl um knapp 2 log Stufen herabgesetzt.

### Ausführunasbeispiel 3: Katheterkontamination bei künstlich infizierter frischer Haut mit und ohne Behandlung mit erfindungsgemäßer Zusammensetzung

Testen eines Keims (V1815 Staph.epidermidis) mit 0,1% Octenidin in 0,85% Carbomergel (+5% Dexpanthenol) an Haut im Schweinehautmodell.

| **Zutaten Gel** | **Hersteller** |
|---|---|
| Carbopol 980 | OTC Pharma, Bönen |
| Natriumhydroxid | ROTH |
| Octenidin(di)hydrochlorid | Caelo |
| Aqua bidest. | |
| Sterile Braunülen G17 weiss | B.Braun |

### Durchführung des Experiments/Ergebnisse:

Frische Haut vom Schwein wurde auf der Oberseite mit etwa 60**µl** Konzentrat von Staph.epidermidis V1815 kontaminiert, indem die Haut in einer Kulturschale auf das Bakterienkonzentrat gelegt wurde. Nach 2 1/2h Inkubation bei 37°C im Brutschrank wurde die Haut auf einen Spezial-Spritzenkasten montiert. Gele auf die Braunülen aufbringen (sowie Braunülen ohne Gel als Kontrolle), durchstechen der Haut mit Kanülen. Kanüle nur 1x duch Haut gestochen (nicht zurückgezogen), Mandrin herausziehen, auf der Rückseite der Haut steril abschneiden, auf Agar bringen und eine intensive Maki-roll (3-4 mal über die Platte rollen mit etwa 50 leichten Impressionen durch Beklopfen) ausführen.

Anschließend wurde alles in den Brutschrank gegeben und für **14h Stunden** inkubiert und ausgezählt. Fotodokumentation. Gruppen siehe Ergebnisse.

| **Name** | **Anzahl Keime** | **Mittelwert** | **Stabw** |
|---|---|---|---|
| K1_14h | 475 | 990,2 | 763,3 |
| K2_14h | 883 | | |
| K3_14h | 1073 | | |
| K4_14h | 2235 | | |
| K5_14h | 285 | | |
| Gel 6 | 6 | 19,2 | 22,6 |
| Gel 7 | 8 | | |
| Gel 8 | 4 | | |
| Gel 9 | 58 | | |
| Gel 10 | 20 | | |

### Fazit/Zusammenfassung:

Die Platte wurde mit dem Zählprogramm (cell explorer) ausgezählt.

Ergebnis:
Durch Verwendung des Gels wurde die Keimzahl um knapp 2 log Stufen herabgesetzt.

### Literatur:

¹Wenzel R and Edmond M. N Engl J Med 2006;355:2781-2783
²Elek SD, Conen PE, Br J Exp Pathol 1957; 38 (6): 573-86
³Hübner NO, Assadian O, Grohmann SA, Diab-Elschahawi M, Kramer A. Efficacy of five alcoholbased skin antiseptics on sebaceous skin used shorter application times the current recommendation of 10 minutes. Eur J Microbiol Infect Dis (2011) 30 : 825-829.
⁴Remijsen. Cell Death and Differentiation (2011) 18, 581-588
⁵N. Chaiyakunapruk, DL Veenstra, BA Lipsky, SD Sullivan, S Saint. Vascular Catheter Site Care: The Clinical and Economic Benefits of Chlorhexidine Gluconate Compared with Povidone lodine. Clinical Infectious Diseases 2003; 37:764-71
⁶Maki, D. G., C. E. Weise, and H. W. Sarafin. 1977. A semiquantitative culture method for identifying intravenous-catheter-related infection. N. Engl. J. Med. 296:1305-1309.

## Patentansprüche

1. Applikator zur perioperativen Desinfektion eines in invasiv erzeugte Körperöffnungen einzuführenden medizinischen Instruments, wobei der Applikator derart ausgebildet ist, dass eine viskose oder schaumartige, antiinfektiöse Zusammensetzung durch eine Relativbewegung zwischen dem Applikator und dem medizinischen Instrument auf eine Oberfläche des medizinischen Instruments aufbringbar ist, **dadurch gekennzeichnet, dass** der Applikator, bevorzugt in Bewegungsrichtung hinten liegend, eine Rückhaltevorrichtung (7) für die antiinfektiöse Zusammensetzung aufweist, wobei der Applikator als im Wesentlichen hohl ausgebildetes Reservoir mit einem, bevorzugt in Bewegungsrichtung hinten liegenden, konisch zulaufenden Abschnitt ausgebildet ist.

2. Applikator nach Anspruch 1, der in einer Innenzone (3) wenigstens ein Reservoir (2) oder wenigstens einen Träger (2) für die antiinfektiöse Zusammensetzung aufweist.

3. Applikator nach Anspruch 1 oder 2, wobei sich an den konischen Abschnitt in Bewegungsrichtung ein zylindrischer Abschnitt anschließt.

4. Applikator nach einem der Ansprüche 1 bis 3, **dadurch gekennzeichnet, dass** der konisch zulaufende oder der zylindrische Abschnitt in Bewegungsrichtung vorne liegend endseitig offen ist.

5. Applikator nach einem der Ansprüche 1 bis 4, wobei die Rückhaltevorrichtung eine Öffnung aufweist, die bevorzugt zusätzlich geschlitzt ist.

6. Applikator nach einem der Ansprüche 1 bis 5, wobei der konisch zulaufende Abschnitt eine Mehrzahl von Schlitzen aufweist, die bevorzugt vom in Bewegungsrichtung hinten liegenden Ende des konischen Abschnitts ausgehen in Axialrichtung ausgebildet sind.

7. Applikator nach einem der Ansprüche 1 bis 6, wobei der konisch zulaufende Abschnitt im Wesentlichen kegelstumpfförmig ist.

8. Applikator nach Anspruch 7, wobei durch die Schlitze eine Anzahl von flexiblen Zungen gebildet wird.

9. Applikator nach einem der Ansprüche 1 bis 8, **dadurch gekennzeichnet, dass** das Reservoir (2) oder der Träger (2) mit einer mit viskosen antiinfektiösen Zusammensetzung gefüllt ist.

10. Applikator nach einem der Ansprüche 1 bis 9, wobei der Applikator seitlich einen in Axialrichtung durchgehenden Schlitz aufweist.

11. Applikator nach einem der Ansprüche 1 bis 10, wobei der Applikator, bevorzugt an dem seitlich in Axialrichtung verlaufenden durchgehenden Schlitz, eine Einführhilfe für das medizinische Instrument aufweist.

12. Applikator nach einem der Ansprüche 1 bis 11, **dadurch gekennzeichnet, dass** er an seiner Außenseite wenigstens zwei Eingriffzonen (1) aufweist.

13. Kit umfassend den Applikator nach einem der Ansprüche 1 bis 12 sowie eine antiinfektiöse Zusammensetzung.

14. Kit nach Anspruch 13, weiter umfassend ein medizinisches Instrument, bevorzugt einen Gefäßkatheter.

15. Verwendung eines Applikators nach einem der Ansprüche 1 bis 12 zum Aufbringen einer viskosen oder schaumartigen mindestens eine antiinfektiöse Verbindung enthaltenden Zusammensetzung auf ein in invasiv erzeugte Körperöffnungen einzuführendes medizinisches Instrument vor Ingebrauchnahme, **dadurch gekennzeichnet, dass** die Zusammensetzung ausgebildet ist, um nach Inkontaktbringen mit dem medizinischen Instrument filmartig auf dem medizinischen Instrument zu haften und dass mindestens ein Teil der filmartig haftenden Zusammensetzung bei Einführung des medizinischen Instruments in den Körper mindestens bis in die Epidermis gelangt.

16. Verfahren zur perioperativen Desinfektion eines in eine invasiv erzeugte Körperöffnung einzuführenden medizinischen Instruments, vorzugsweise eines Gefäßkatheters, und/oder eines durch die Einführung eines medizinischen Instruments in den Körper erzeugten Stichkanals, umfassend Aufbringen einer viskosen oder schaumartigen, mindestens eine antiinfektiöse Verbindung enthaltenden Zusammensetzung auf das medizinische Instrument vor Ingebrauchnahme, dadurch charakterisiert dass die Zusammensetzung ausgebildet ist, um nach Inkontaktbringen mit dem medizinischen Instrument filmartig auf dem medizinischen Instrument zu haften, dass mindestens ein Teil der filmartig haftenden Zusammensetzung bei Einführung des medizinischen Instruments in den Körper mindestens bis in die Epidermis gelangt und dass das Aufbringen mittels eines Applikators aus einem der Ansprüche 1 bis 12 erfolgt.

## Claims

1. Applicator for the perioperative disinfection of a medical instrument to be inserted into invasively generated body openings, wherein the applicator is designed in such a way that a viscous or foam-like, anti-infectious composition can be applied to a surface of the medical instrument by a relative movement between the applicator and the medical instrument, **characterised in that** the applicator, which is preferably located at the rear in the direction of movement, comprises a retaining device (7) for the anti-infectious composition, wherein the applicator is formed as an substantially hollow reservoir with a conically tapering section which is preferably located at the rear in the direction of movement.

2. Applicator according to claim 1, which comprises in an interior zone (3) at least one reservoir (2) or at least one carrier (2) for the anti-infectious composition.

3. Applicator according to claim 1 or 2, wherein a cylindrical section adjoins the conical section in the direction of movement.

4. Applicator according to one of claims 1 to 3, **characterised in that** the conically tapering section or the cylindrical section is open at the front end, as viewed in the direction of movement.

5. Applicator according to one of claims 1 to 4, wherein the retaining device has an opening which is preferably additionally slotted.

6. Applicator according to one of claims 1 to 5, wherein the conically tapering section has a plurality of slots, which are preferably formed in the axial direction starting from the rear end of the conical section, as viewed in the direction of movement.

7. Applicator according to one of claims 1 to 5, wherein the conically tapering section is substantially frustum-shaped.

8. Applicator according to claim 7, wherein a plurality of flexible tongues are formed by the slots.

9. Applicator according to one of claims 1 to 8, **characterised in that** the reservoir (2) or the carrier (2) is filled with a viscous, anti-infectious composition.

10. Applicator according to one of claims 1 to 9, wherein the applicator has a lateral slot which is continuous in the axial direction.

11. Applicator according to one of claims 1 to 10, wherein the applicator preferably has an insertion aid for the medical instrument at the continuous slot extending laterally in the axial direction.

12. Applicator according to one of claims 1 to 11, **characterised in that** it has at least two engagement zones (1) on its exterior.

13. Kit comprising the applicator according to one of claims 1 to 12 as well as an anti-infectious composition.

14. Kit according to claim 13, further comprising a medical instrument, preferably a vascular catheter.

15. Use of an applicator according to one of claims 1 to 12 for application of a viscous or foam-like composition containing at least one anti-infectious compound to a medical instrument to be inserted into invasively generated body openings prior to use, **characterised in that** the composition is designed to adhere to the medical instrument like a film after being brought into contact with the medical instrument, and **in that** at least a part of the composition adhering like a film reaches at least to the epidermis when the medical instrument is inserted into the body.

16. Method for the perioperative disinfection of a medical instrument to be inserted into an invasively generated body opening, preferably a vascular catheter, and/or an injection channel generated by the insertion of a medical instrument into the body, comprising the application of a viscous or foam-like composition containing at least one anti-infectious compound to the medical instrument prior to use, **characterised in that** the composition is designed to adhere to the medical instrument like a film after being brought into contact with the medical instrument, **in that** at least a part of the composition adhering like a film reaches at least into the epidermis when the medical instrument is inserted into the body, and **in that** the application takes place by means of an applicator according to one of claims 1 to 12.

## Revendications

1. Applicateur permettant la désinfection péri-opératoire d'un instrument médical destiné à être introduit dans des ouvertures corporelles obtenues de manière invasive, cet applicateur étant réalisé de façon à permettre d'appliquer une composition anti-infectieuse, visqueuse ou en forme de mousse sur la surface de l'instrument médical par un déplacement relatif entre l'applicateur et cet instrument,
**caractérisé en ce que**
l'applicateur comporte un dispositif de retenue (7) de la composition anti-infectieuse, de préférence situé à l'arrière dans la direction de déplacement, l'applicateur étant réalisé sous la forme d'un réservoir essentiellement creux comportant un segment s'étendant coniquement situé de préférence à l'arrière dans la direction de déplacement.

2. Applicateur conforme à la revendication 1,
qui comporte, dans une zone interne (3), au moins un réservoir (2) ou au moins un support (2) pour la composition anti-infectieuse.

3. Applicateur conforme à la revendication 1 ou 2,
dans lequel un segment cylindrique se connecte au segment conique dans la direction de déplacement.

4. Applicateur conforme à l'une des revendications 1 à 3,
**caractérisé en ce que**
le segment s'étendant coniquement ou le segment cylindrique est ouvert à son extrémité située à l'avant dans la direction de déplacement.

5. Applicateur conforme à l'une des revendications 1 à 4,
dans lequel le dispositif de retenue comporte une ouverture qui est en outre de préférence fendue.

6. Applicateur conforme à l'une des revendications 1 à 5,
dans lequel le segment s'étendant coniquement comporte un ensemble de fentes qui sont de préférence formées en direction axiale à partir de l'extrémité du segment conique située à l'arrière dans la direction de déplacement.

7. Applicateur conforme à l'une des revendications 1 à 6,
dans lequel le segment s'étendant coniquement est essentiellement en forme de tronc de cône.

8. Applicateur conforme à la revendication 7,
dans lequel un ensemble de languettes flexibles est formé par les fentes.

9. Applicateur conforme à l'une des revendications 1 à 8,
**caractérisé en ce que**
le réservoir (2) ou le support (2) est rempli d'une composition anti-infectieuse, visqueuse.

10. Applicateur conforme à l'une des revendications 1 à 9,
comportant latéralement une fente traversante en direction axiale.

11. Applicateur conforme à l'une des revendications 1 à 10,
comprenant un moyen aidant l'introduction de l'instrument médical de préférence sur la fente traversante s'étendant latéralement en direction axiale.

12. Applicateur conforme à l'une des revendications 1 à 11,
**caractérisé en ce qu'**
il comporte au moins deux dômes de préhension (1) sur sa face externe.

13. Kit comprenant un applicateur conforme à l'une des revendications 1 à 12, ainsi qu'une composition anti-infectieuse.

14. Kit conforme à la revendication 13,
comprenant en outre un instrument médical, de préférence un cathéter vasculaire.

15. Utilisation d'un applicateur conforme à l'une des revendications 1 à 12, pour appliquer une composition visqueuse ou en forme de mousse renfermant au moins un composé anti-infectieux, sur un instrument médical destiné à être introduit dans des ouvertures corporelles produites de manière invasive, avant son utilisation,
**caractérisé en ce que**
la composition est réalisée de façon à adhérer sous la forme d'un film sur l'instrument médical, après sa mise en contact avec celui-ci, et, lors de l'introduction de l'instrument médical dans le corps, au moins une partie de la composition adhérant sous la forme d'un film parvient au moins jusqu'à l'épiderme.

16. Procédé de désinfection péri-opératoire d'un instrument médical, de préférence d'un cathéter vasculaire destiné à être introduit dans une ouverture du corps produite de manière invasive, et/ou d'un canal de perçage produit par l'introduction d'un instrument médical dans le corps, comprenant des étapes consistant à appliquer une composition visqueuse ou sous forme de mousse renfermant au moins un composé anti-infectieux sur l'instrument médical avant son utilisation,
**caractérisé en ce que**
la composition est réalisée pour adhérer sous la forme d'un film sur l'instrument médical après sa mise en contact avec celui-ci, au moins une partie de la composition adhérant sous la forme d'un film parvient au moins jusqu'à l'épiderme lors de l'introduction de l'instrument médical dans le corps, et l'application est effectuée au moyen d'un applicateur conforme à l'une des revendications 1 à 12.
